# EUROPÄISCHE PATENTSCHRIFT

(11) **EP 3 132 041 B1**
(45) Veröffentlichungstag und Bekanntmachung des Hinweises auf die Patenterteilung: **18.03.2020**
(21) Anmeldenummer: 15717456.6
(22) Anmeldetag: 13.04.2015
(51) Int. Cl.: C12N 15/86, A61K 48/00

(54) **AAV-VEKTOREN FÜR VASKULÄRE GENTHERAPIE IN KORONARER HERZERKRANKUNG UND PERIPHERER ISCHÄMIE**
AAV VECTORS FOR VASCULAR GENE THERAPY IN CORONARY HEART DISEASE AND PERIPHERAL ISCHAEMIA
VECTEURS AAV POUR THÉRAPIE GÉNIQUE VASCULAIRE DE MALADIES CARDIAQUES CORONARIENNES ET DE L'ISCHÉMIE PÉRIPHÉRIQUE

(30) Priorität: 14.04.2014 DE 102014207153
(43) Veröffentlichungstag der Anmeldung: 22.02.2017
(73) Patentinhaber: Kupatt, Christian, 81366 München (DE); Hinkel, Rabea, 80796 München (DE)
(72) Erfinder: Kupatt, Christian, 81366 München (DE); Hinkel, Rabea, 80796 München (DE)
(74) Vertreter: Grund, Martin
(86) Internationale Anmeldenummer: PCT/EP2015/057987
(87) Internationale Veröffentlichungsnummer: WO 2015/158667

(56) Entgegenhaltungen:
- HINKEL R ET AL: "P596:Therapeutic neovascularization by AAV2/9-based gene transfer of Thymosin B4: results of a preclinical pig study", CARDIOVASCULAR RESEARCH, Bd. 93, Nr. Suppl. 1, 15. März 2012 (2012-03-15), Seite S117, XP055202679,
- HINKEL R ET AL: "MRTF-A controls vessel growth and maturation by increasing the expression of CCN1 and CCN2", NATURE COMMUNICATIONS, Bd. 5, 9. Juni 2014 (2014-06-09), Seiten 1-9, XP055202593, DOI: 10.1038/ncomms4970
- MIKHAILOV A T ET AL: "In Search of Novel Targets for Heart Disease: Myocardin and Myocardin-Related Transcriptional Cofactors", BIOCHEMISTRY RESEARCH INTERNATIONAL, Bd. 6, Nr. 10, 1. Januar 2012 (2012-01-01) , Seiten 1-11, XP055202587, ISSN: 2090-2247, DOI: 10.1161/ATVBAHA.110.218149
- KUPATT C ET AL: "Cotransfection of Vascular Endothelial Growth Factor-A and Platelet-Derived Growth Factor-B Via Recombinant Adeno-Associated Virus Resolves Chronic Ischemic Malperfusion", JOURNAL OF THE AMERICAN COLLEGE OF CARDIOLOGY, ELSEVIER, NEW YORK, NY, US, Bd. 56, Nr. 5, 27. Juli 2010 (2010-07-27), Seiten 414-422, XP027160198, ISSN: 0735-1097 [gefunden am 2010-07-20]

<110> KUPATT, Christian HINKEL, Rabea
<120> AAV-VEKTOREN FÜR VASKULÄRE GENTHERAPIE IN KORONARER HERZERKRANKUNG UND PERIPHERER ISCHÄMIE
<130> 233-001DE
<150> DE 10 2014 207 153.4
   <151> 2014-04-14
<160> 17
<170> PatentIn version 3.5
<210> 1
   <211> 69
   <212> RNA
   <213> Künstliche Sequenz
<400> 1
   gauccccgca uggagcuggu ggagaagaau ucaagagauu cuucuccacc agcuccaugu 60
   uuuuggaaa 69
<210> 2
   <211> 23
   <212> DNA
   <213> Künstliche Sequenz
<400> 2
   tctagttgcc agccatctgt tgt 23
<210> 3
   <211> 18
   <212> DNA
   <213> Künstliche Sequenz
<400> 3
   tgggagtggc accttcca 18
<210> 4
   <211> 20
   <212> DNA
   <213> Künstliche Sequenz
<400> 4
   aattcaacgg cacagtcaag 20
<210> 5
   <211> 20
   <212> DNA
   <213> Künstliche Sequenz
<400> 5
   atggtggtga agacaccagt 20
<210> 6
   <211> 21
   <212> DNA
   <213> Künstliche Sequenz
<400> 6
   tcatcgatat gtctgacaaa c 21
<210> 7
   <211> 21
   <212> DNA
   <213> Künstliche Sequenz
<400> 7
   cagcttgctt ctcttgttca a 21
<210> 8
   <211> 24
   <212> DNA
   <213> Künstliche Sequenz
<400> 8
   aatccatggg tcgacggtat cgat 24
<210> 9
   <211> 24
   <212> DNA
   <213> Künstliche Sequenz
<400> 9
   ataccatggt caggcaccgg gctt 24
<210> 10
   <211> 20
   <212> DNA
   <213> Künstliche Sequenz
<400> 10
   gctaaacaac tcaacgagga 20
<210> 11
   <211> 21
   <212> DNA
   <213> Künstliche Sequenz
<400> 11
   ggctgcaact gcgctcctct g 21
<210> 12
   <211> 19
   <212> DNA
   <213> Künstliche Sequenz
<400> 12
   ccctagctgc ctaccgact 19
<210> 13
   <211> 23
   <212> DNA
   <213> Künstliche Sequenz
<400> 13
   cattccacag gtcttagaac agg 23
<210> 14
   <211> 20
   <212> DNA
   <213> Künstliche Sequenz
<400> 14
   tcgaatacga tgactcggtg 20
<210> 15
   <211> 19
   <212> DNA
   <213> Künstliche Sequenz
<400> 15
   gtttgtccct atttctatc 19
<210> 16
   <211> 7890
   <212> DNA
   <213> Künstliche Sequenz
<400> 16
   agcgcccaat acgcaaaccg cctctccccg cgcgttggcc gattcattaa tgcagctggc      60
   acgacaggtt tcccgactgg aaagcgggca gtgagcgcaa cgcaattaat gtgagttagc     120
   tcactcatta ggcaccccag gctttacact ttatgcttcc ggctcgtatg ttgtgtggaa     180
   ttgtgagcgg ataacaattt cacacaggaa acagctatga ccatgattac gccagattta     240
   attaaggctg cgcgctcgct cgctcactga ggccgcccgg gcaaagcccg ggcgtcgggc     300
   gacctttggt cgcccggcct cagtgagcga gcgagcgcgc agagagggag tggccaactc     360
   catcactagg ggttccttgt agttaatgat taacccgcca tgctacttat ctacgtagcc     420
   atgctctagg aagatcggaa ttcgccctta agctagctag ttattaatag taatcaatta     480
   cggggtcatt agttcatagc ccatatatgg agttccgcgt tacataactt acggtaaatg     540
   gcccgcctgg ctgaccgccc aacgaccccc gcccattgac gtcaataatg acgtatgttc     600
   ccatagtaac gccaataggg actttccatt gacgtcaatg ggtggagtat ttacggtaaa     660
   ctgcccactt ggcagtacat caagtgtatc atatgccaag tacgccccct attgacgtca     720
   atgacggtaa atggcccgcc tggcattatg cccagtacat gaccttatgg gactttccta     780
   cttggcagta catctacgta ttagtcatcg ctattaccat ggtgatgcgg ttttggcagt     840
   acatcaatgg gcgtggatag cggtttgact cacggggatt tccaagtctc caccccattg     900
   acgtcaatgg gagtttgttt tggcaccaaa atcaacggga ctttccaaaa tgtcgtaaca 960
   actccgcccc attgacgcaa atgggcggta ggcgtgtacg gtgggaggtc tatataagca 1020
   gagctggttt agtgaaccgt cagatcctgc agaagttggt cgtgaggcac tgggcaggta 1080
   agtatcaagg ttacaagaca ggtttaagga gaccaataga aactgggctt gtcgagacag 1140
   agaagactct tgcgtttctg ataggcacct attggtctta ctgacatcca ctttgccttt 1200
   ctctccacag gtgtccaggc ggccgctcaa acagacacca taacatgctg cccccttccg 1260
   tcattgctgt gaatgggctg gacggaggag gggctggcga aaatgacgac gagccagtgc 1320
   tcctgtctct gtctgcggcc cccagccccc agagcgaagc tgttgccaat gaactgcagg 1380
   agctgtccct gcagcccgag ctgactctag gcctccatcc tgggaggaac cccaatttac 1440
   ctccacttag tgagcggaag aatgtgctgc agttgaagct ccagcagcgg cggacccggg 1500
   aggaactggt gagccaaggg atcatgccgc ctttgaaaag ccccgctgca tttcatgagc 1560
   agagaagaag cctggagcgg gccaggaccg aggactattt gaaacggaag atccgttccc 1620
   ggcccgagag agcagagctg gtcaggatgc acattctgga agagacctcg gctgagcctt 1680
   cgctccaggc caagcagctg aagctgaaga gagccaggct ggctgatgac ctcaatgaaa 1740
   agattgcaca gaggcctggc cccatggagc tggtggagaa gaatatcctg cctgtggagt 1800
   ccagcctgaa ggaggctatc attgtgggcc aggtaaatta cccaaaggta gcagacagtt 1860
   cctccttcga cgaggacagc agcgatgccc tgtctcctga gcagcctgcc agccatgagt 1920
   cccagggttc agtgccatca cccttggagt cccgagtcag tgatccactg cccagtgcca 1980
   cctccatatc acccactcag gttctttctc aactcccaat ggctccggat cctggagaga 2040
   cgctttttct ggcagagcag cctcctctgc ctcccgcacc tctgctgccc ccaagcctag 2100
   ccaatggaag catcgtcccc actgccaagc ctgctcccac actcatcaag caaagccaac 2160
   ccaagtctgc cagcgagaaa tcacagcgca gcaagaaggc caaggagctg aagccaaagg 2220
   tgaagaagct caagtaccac cagtacatcc ccccggacca gaagcaggac aagggggcgc 2280
   ccgccatgga ctcctcctat gccaagatcc tgcagcagca gcagctcttc ctgcagctgc 2340
   agatcctcaa ccagcagcag cagcagcagc agcaacagca ctacaactac caggccatcc 2400
   tgcctgcccc tcccaagccc tcggctgaga ctcctggaag cagtgcccct accccatcac 2460
   gcagcctctc caccagtagc agccccagct caggcacccc agggcccagc gggctggcac 2520
   gccagagcag caccgcacta gctgccaaac caggagccct gccagccaac ctggatgaca 2580
   tgaaggtggc agagctgaag caagaactga agttgcggtc ccttcccgtc tcaggcacca 2640
   agacagagct gatagaacgc ctgcgtgcct accaagacca agtcagccca gctccaggag 2700
   cccccaaggc ccctgccacc acctctgtgc tgtccaaggc tggtgaggta gtggtcgcct 2760
   tccctgcggc cctgctaagc acagggtcag ctcttgtaac agcaggcctt gcaccagctg 2820
   agatggtggt ggccacagta accagcaatg gcatggtgaa gtttggcagc acaggctcca 2880
   caccccccgt gtctcccacc ccttcagagc gctcactgct cagcacgggt gatgagaatt 2940
   ccacacctgg ggatgccttt ggtgaaatgg tgacatcgcc gctgacacag ctcaccctgc 3000
   aggcctcccc actgcagatc gtgaaggagg agggtgcccg tgctgcgtcc tgctgtctaa 3060
   gccctggtgc tcgggctgag ctggagggac tggacaagga ccagatgctg caggagaagg 3120
   acaagcagat tgaggagctg acccgaatgc tccaacagaa gcagcagctg gttgagctgc 3180
   tgcggctaca gctggagcag cagaagcggg cccagcagcc agccccagcc agcagccctg 3240
   tgaagaggga aagtggtttc tccagttgcc agctgagctg ccagccccag ggctctgccc 3300
   atgcttttgg ctctggccta gtggttccca ctaccaacca tggagacact caggccccag 3360
   cgccagagtc cccacctgtg gtggtgaagc aggaagctgg gccacctgag ccagatctgg 3420
   cccccagctc ccagctgctc ttgggctccc agggcaccag cttcctcaag agggtcagcc 3480
   ctcctaccct ggtcactgac tctacaggga ctcacctcat cctcactgtg accaataaga 3540
   gtgctgatgg ccctggcttg cctgcaggga gcccccagca gcccttgtcc cagcctggtt 3600
   ctccagcccc tggtccacct gcccagatgg acctggagca cccacctcag cctccgtttg 3660
   caacccccac atctctgctg aagaaggagc cccctggtta tgaagagact gtgacccagc 3720
   agcctaagca gcaggaaaat ggctcctcca gtcagcacat ggatgatctg tttgatattc 3780
   ttattcagag tggagagatt tcagcagatt tcaaagagcc accatcccta ccaggcaagg 3840
   aaaagtcacc tccagcagca gcagcgtatg ggcctccatt gacaccacaa ccctcgcctt 3900
   tgagtgaact cccccaagct gctcctccac caggttcccc caccctccca gggcgccttg 3960
   aagacttcct ggagagcagc acagggctgc ccctgctgac aagtgggcac gagggaccag 4020
   aacccctttc cctcattgat gacctccaca gccagatgct gagcagctcc gccatcctgg 4080
   accacccccc atcacccatg gacacctctg aattgcactt tgctcctgag cccagcagtg 4140
   gtatgggcct ggacctggct gttggccacc tggacagcat ggactggctg gagctgtcgt 4200
   ctggtggccc tgtgctcagc ctggctcccc tcagcactgc agcccccagc ctcttctcga 4260
   tggacttcct ggatggacac gacttgcagc tccactggga ttcctgcttg tacccatacg 4320
   acgtgccaga ctagaagctt ggatccaatc aacctctgga ttacaaaatt tgtgaaagat 4380
   tgactggtat tcttaactat gttgctcctt ttacgctatg tggatacgct gctttaatgc 4440
   ctttgtatca tgctattgct tcccgtatgg ctttcatttt ctcctccttg tataaatcct 4500
   ggttgctgtc tctttatgag gagttgtggc ccgttgtcag gcaacgtggc gtggtgtgca 4560
   ctgtgtttgc tgacgcaacc cccactggtt ggggcattgc caccacctgt cagctccttt 4620
   ccgggacttt cgctttcccc ctccctattg ccacggcgga actcatcgcc gcctgccttg 4680
   cccgctgctg gacaggggct cggctgttgg gcactgacaa ttccgtggtg ttgtcgggga 4740
   agctgacgtc ctttccatgg ctgctcgcct gtgttgccac ctggattctg cgcgggacgt 4800
   ccttctgcta cgtcccttcg gccctcaatc cagcggacct tccttcccgc ggcctgctgc 4860
   cggctctgcg gcctcttccg cgtcttcgag atctgcctcg actgtgcctt ctagttgcca 4920
   gccatctgtt gtttgcccct cccccgtgcc ttccttgacc ctggaaggtg ccactcccac 4980
   tgtcctttcc taataaaatg aggaaattgc atcgcattgt ctgagtaggt gtcattctat 5040
   tctggggggt ggggtggggc aggacagcaa gggggaggat tgggaagaca atagcaggca 5100
   tgctggggac tcgagttaag ggcgaattcc cgattaggat cttcctagag catggctacg 5160
   tagataagta gcatggcggg ttaatcatta actacaagga acccctagtg atggagttgg 5220
   ccactccctc tctgcgcgct cgctcgctca ctgaggccgg gcgaccaaag gtcgcccgac 5280
   gcccgggctt tgcccgggcg gcctcagtga gcgagcgagc gcgcagcctt aattaaccta 5340
   attcactggc cgtcgtttta caacgtcgtg actgggaaaa ccctggcgtt acccaactta 5400
   atcgccttgc agcacatccc cctttcgcca gctggcgtaa tagcgaagag gcccgcaccg 5460
   atcgcccttc ccaacagttg cgcagcctga atggcgaatg ggacgcgccc tgtagcggcg 5520
   cattaagcgc ggcgggtgtg gtggttacgc gcagcgtgac cgctacactt gccagcgccc 5580
   tagcgcccgc tcctttcgct ttcttccctt cctttctcgc cacgttcgcc ggctttcccc 5640
   gtcaagctct aaatcggggg ctccctttag ggttccgatt tagtgcttta cggcacctcg 5700
   accccaaaaa acttgattag ggtgatggtt cacgtagtgg gccatcgccc cgatagacgg 5760
   tttttcgccc tttgacgctg gagttcacgt tcctcaatag tggactcttg ttccaaactg 5820
   gaacaacact caaccctatc tcggtctatt cttttgattt ataagggatt tttccgattt 5880
   cggcctattg gttaaaaaat gagctgattt aacaaaaatt taacgcgaat tttaacaaaa 5940
   tattaacgtt tataatttca ggtggcatct ttcggggaaa tgtgcgcgga acccctattt 6000
   gtttattttt ctaaatacat tcaaatatgt atccgctcat gagacaataa ccctgataaa 6060
   tgcttcaata atattgaaaa aggaagagta tgagtattca acatttccgt gtcgccctta 6120
   ttcccttttt tgcggcattt tgccttcctg tttttgctca cccagaaacg ctggtgaaag 6180
   taaaagatgc tgaagatcag ttgggtgcac gagtgggtta catcgaactg gatctcaata 6240
   gtggtaagat ccttgagagt tttcgccccg aagaacgttt tccaatgatg agcactttta 6300
   aagttctgct atgtggcgcg gtattatccc gtattgacgc cgggcaagag caactcggtc 6360
   gccgcataca ctattctcag aatgacttgg ttgagtactc accagtcaca gaaaagcatc 6420
   ttacggatgg catgacagta agagaattat gcagtgctgc cataaccatg agtgataaca 6480
   ctgcggccaa cttacttctg acaacgatcg gaggaccgaa ggagctaacc gcttttttgc 6540
   acaacatggg ggatcatgta actcgccttg atcgttggga accggagctg aatgaagcca 6600
   taccaaacga cgagcgtgac accacgatgc ctgtagtaat ggtaacaacg ttgcgcaaac 6660
   tattaactgg cgaactactt actctagctt cccggcaaca attaatagac tggatggagg 6720
   cggataaagt tgcaggacca cttctgcgct cggcccttcc ggctggctgg tttattgctg 6780
   ataaatctgg agccggtgag cgtgggtctc gcggtatcat tgcagcactg gggccagatg 6840
   gtaagccctc ccgtatcgta gttatctaca cgacggggag tcaggcaact atggatgaac 6900
   gaaatagaca gatcgctgag ataggtgcct cactgattaa gcattggtaa ctgtcagacc 6960
   aagtttactc atatatactt tagattgatt taaaacttca tttttaattt aaaaggatct 7020
   aggtgaagat cctttttgat aatctcatga ccaaaatccc ttaacgtgag ttttcgttcc 7080
   actgagcgtc agaccccgta gaaaagatca aaggatcttc ttgagatcct ttttttctgc 7140
   gcgtaatctg ctgcttgcaa acaaaaaaac caccgctacc agcggtggtt tgtttgccgg 7200
   atcaagagct accaactctt tttccgaagg taactggctt cagcagagcg cagataccaa 7260
   atactgtcct tctagtgtag ccgtagttag gccaccactt caagaactct gtagcaccgc 7320
   ctacatacct cgctctgcta atcctgttac cagtggctgc tgccagtggc gataagtcgt 7380
   gtcttaccgg gttggactca agacgatagt taccggataa ggcgcagcgg tcgggctgaa 7440
   cggggggttc gtgcacacag cccagcttgg agcgaacgac ctacaccgaa ctgagatacc 7500
   tacagcgtga gctatgagaa agcgccacgc ttcccgaagg gagaaaggcg gacaggtatc 7560
   cggtaagcgg cagggtcgga acaggagagc gcacgaggga gcttccaggg ggaaacgcct 7620
   ggtatcttta tagtcctgtc gggtttcgcc acctctgact tgagcgtcga tttttgtgat 7680
   gctcgtcagg ggggcggagc ctatggaaaa acgccagcaa cgcggccttt ttacggttcc 7740
   tggccttttg ctgcggtttt gctcacatgt tctttcctgc gttatcccct gattctgtgg 7800
   ataaccgtat taccgccttt gagtgagctg ataccgctcg ccgcagccga acgaccgagc 7860
   gcagcgagtc agtgagcgag gaagcggaag 7890
<210> 17
   <211> 7596
   <212> DNA
   <213> Künstliche Sequenz
<400> 17
   agcgcccaat acgcaaaccg cctctccccg cgcgttggcc gattcattaa tgcagctggc      60
   acgacaggtt tcccgactgg aaagcgggca gtgagcgcaa cgcaattaat gtgagttagc     120
   tcactcatta ggcaccccag gctttacact ttatgcttcc ggctcgtatg ttgtgtggaa     180
   ttgtgagcgg ataacaattt cacacaggaa acagctatga ccatgattac gccagattta     240
   attaaggctg cgcgctcgct cgctcactga ggccgcccgg gcaaagcccg ggcgtcgggc     300
   gacctttggt cgcccggcct cagtgagcga gcgagcgcgc agagagggag tggccaactc 360
   catcactagg ggttccttgt agttaatgat taacccgcca tgctacttat ctacgtagcc 420
   atgctctagg aagatcggaa ttcgccctta agctagctag ttattaatag taatcaatta 480
   cggggtcatt agttcatagc ccatatatgg agttccgcgt tacataactt acggtaaatg 540
   gcccgcctgg ctgaccgccc aacgaccccc gcccattgac gtcaataatg acgtatgttc 600
   ccatagtaac gccaataggg actttccatt gacgtcaatg ggtggagtat ttacggtaaa 660
   ctgcccactt ggcagtacat caagtgtatc atatgccaag tacgccccct attgacgtca 720
   atgacggtaa atggcccgcc tggcattatg cccagtacat gaccttatgg gactttccta 780
   cttggcagta catctacgta ttagtcatcg ctattaccat ggtgatgcgg ttttggcagt 840
   acatcaatgg gcgtggatag cggtttgact cacggggatt tccaagtctc caccccattg 900
   acgtcaatgg gagtttgttt tggcaccaaa atcaacggga ctttccaaaa tgtcgtaaca 960
   actccgcccc attgacgcaa atgggcggta ggcgtgtacg gtgggaggtc tatataagca 1020
   gagctggttt agtgaaccgt cagatcctgc agaagttggt cgtgaggcac tgggcaggta 1080
   agtatcaagg ttacaagaca ggtttaagga gaccaataga aactgggctt gtcgagacag 1140
   agaagactct tgcgtttctg ataggcacct attggtctta ctgacatcca ctttgccttt 1200
   ctctccacag gtgtccaggc ggccgctcaa acagacacca taacatgccg cctttgaaaa 1260
   gtccagccgc atttcatgag cagagaagga gcttggagcg ggccaggaca gaggactatc 1320
   tcaaacggaa gattcgttcc cggccggaga gatcggagct ggtcaggatg cacattttgg 1380
   aagagacctc ggctgagcca tccctccagg ccaagcagct gaagctgaag agagccagac 1440
   tagccgatga cctcaatgag aagattgcac agaggcctgg ccccatggag ctggtggaga 1500
   agaacatcct tcctgttgag tccagcctga aggaagccat cattgtgggc caggtgaact 1560
   atcccaaagt agcagacagc tcttccttcg atgaggacag cagcgatgcc ttatcccccg 1620
   agcagcctgc cagccatgag tcccagggtt ctgtgccgtc acccctggag gcccgagtca 1680
   gcgaaccact gctcagtgcc acctctgcat cccccaccca ggttgtgtct caacttccga 1740
   tgggccggga ttccagagaa atgcttttcc tggcagagca gcctcctctg cctcccccac 1800
   ctctgctgcc tcccagcctc accaatggaa ccactatccc cactgccaag tccaccccca 1860
   cactcattaa gcaaagccaa cccaagtctg ccagtgagaa gtcacagcgc agcaagaagg 1920
   ccaaggagct gaagccaaag gtgaagaagc tcaagtacca ccagtacatc cccccggacc 1980
   agaagcagga caggggggca ccccccatgg actcatccta cgccaagatc ctgcagcagc 2040
   agcagctctt cctccagctg cagatcctca accagcagca gcagcagcac cacaactacc 2100
   aggccatcct gcctgccccg ccaaagtcag caggcgaggc cctgggaagc agcgggaccc 2160
   ccccagtacg cagcctctcc actaccaata gcagctccag ctcgggcgcc cctgggccct 2220
   gtgggctggc acgtcagaac agcacctcac tgactggcaa gccgggagcc ctgccggcca 2280
   acctggacga catgaaggtg gcagagctga agcaggagct gaagttgcga tcactgcctg 2340
   tctcgggcac caaaactgag ctgattgagc gccttcgagc ctatcaagac caaatcagcc 2400
   ctgtgccagg agcccccaag gcccctgccg ccacctctat cctgcacaag gctggcgagg 2460
   tggtggtagc cttcccagcg gcccggctga gcacggggcc agccctggtg gcagcaggcc 2520
   tggctccagc tgaggtggtg gtggccacgg tggccagcag tggggtggtg aagtttggca 2580
   gcacgggctc cacgcccccc gtgtctccca ccccctcgga gcgctcactg ctcagcacgg 2640
   gcgatgaaaa ctccaccccc ggggacacct ttggtgagat ggtgacatca cctctgacgc 2700
   agctgaccct gcaggcctcg ccactgcaga tcctcgtgaa ggaggagggc ccccgggccg 2760
   ggtcctgttg cctgagccct gggggcgggg cggagctaga ggggcgcgac aaggaccaga 2820
   tgctgcagga gaaagacaag cagatcgagg cgctgacgcg catgctccgg cagaagcagc 2880
   agctggtgga gcggctcaag ctgcagctgg agcaggagaa gcgagcccag cagcccgccc 2940
   ccgcccccgc ccccctcggc acccccgtga agcaggagaa cagcttctcc agctgccagc 3000
   tgagccagca gcccctgggc cccgctcacc cattcaaccc cagcctggcg gccccagcca 3060
   ccaaccacat agacccttgt gctgtggccc cggggccccc gtccgtggtg gtgaagcagg 3120
   aagccttgca gcctgagccc gagccggtcc ccgcccccca gttgcttctg gggcctcagg 3180
   gccccagcct catcaagggg gttgcacctc ccaccctcat caccgactcc acagggaccc 3240
   accttgtcct caccgtgacc aataagaatg cagacagccc tggcctgtcc agtgggagcc 3300
   cccagcagcc ctcgtcccag cctggctctc cagcgcctgc cccctctgcc cagatggacc 3360
   tggagcaccc actgcagccc ctctttggga cccccacttc tctgctgaag aaggaaccac 3420
   ctggctatga ggaagccatg agccagcagc ccaaacagca ggaaaatggt tcctcaagcc 3480
   agcagatgga cgacctgttt gacattctca ttcagagcgg agaaatttca gcagatttca 3540
   aggagccgcc atccctgcca gggaaggaga agccatcccc gaagacagtc tgtgggtccc 3600
   ccctggcagc acagccatca ccttctgctg agctccccca ggctgcccca cctcctccag 3660
   gctcaccctc cctccctgga cgcctggagg acttcctgga gagcagcacg gggctgcccc 3720
   tgctgaccag tgggcatgac gggccagagc ccctttccct cattgacgac ctccatagcc 3780
   agatgctgag cagcactgcc atcctggacc accccccgtc acccatggac acctcggaat 3840
   tgcactttgt tcctgagccc agcagcacca tgggcctgga cctggctgat ggccacctgg 3900
   acagcatgga ctggctggag ctgtcgtcag gtggtcccgt gctgagccta gcccccctca 3960
   gcaccacagc ccccagcctc ttctccacag acttcctcga tggccatgat ttgcagctgc 4020
   actgggattc ctgcttgtag aagcttggat ccaatcaacc tctggattac aaaatttgtg 4080
   aaagattgac tggtattctt aactatgttg ctccttttac gctatgtgga tacgctgctt 4140
   taatgccttt gtatcatgct attgcttccc gtatggcttt cattttctcc tccttgtata 4200
   aatcctggtt gctgtctctt tatgaggagt tgtggcccgt tgtcaggcaa cgtggcgtgg 4260
   tgtgcactgt gtttgctgac gcaaccccca ctggttgggg cattgccacc acctgtcagc 4320
   tcctttccgg gactttcgct ttccccctcc ctattgccac ggcggaactc atcgccgcct 4380
   gccttgcccg ctgctggaca ggggctcggc tgttgggcac tgacaattcc gtggtgttgt 4440
   cggggaagct gacgtccttt ccatggctgc tcgcctgtgt tgccacctgg attctgcgcg 4500
   ggacgtcctt ctgctacgtc ccttcggccc tcaatccagc ggaccttcct tcccgcggcc 4560
   tgctgccggc tctgcggcct cttccgcgtc ttcgagatct gcctcgactg tgccttctag 4620
   ttgccagcca tctgttgttt gcccctcccc cgtgccttcc ttgaccctgg aaggtgccac 4680
   tcccactgtc ctttcctaat aaaatgagga aattgcatcg cattgtctga gtaggtgtca 4740
   ttctattctg gggggtgggg tggggcagga cagcaagggg gaggattggg aagacaatag 4800
   caggcatgct ggggactcga gttaagggcg aattcccgat taggatcttc ctagagcatg 4860
   gctacgtaga taagtagcat ggcgggttaa tcattaacta caaggaaccc ctagtgatgg 4920
   agttggccac tccctctctg cgcgctcgct cgctcactga ggccgggcga ccaaaggtcg 4980
   cccgacgccc gggctttgcc cgggcggcct cagtgagcga gcgagcgcgc agccttaatt 5040
   aacctaattc actggccgtc gttttacaac gtcgtgactg ggaaaaccct ggcgttaccc 5100
   aacttaatcg ccttgcagca catccccctt tcgccagctg gcgtaatagc gaagaggccc 5160
   gcaccgatcg cccttcccaa cagttgcgca gcctgaatgg cgaatgggac gcgccctgta 5220
   gcggcgcatt aagcgcggcg ggtgtggtgg ttacgcgcag cgtgaccgct acacttgcca 5280
   gcgccctagc gcccgctcct ttcgctttct tcccttcctt tctcgccacg ttcgccggct 5340
   ttccccgtca agctctaaat cgggggctcc ctttagggtt ccgatttagt gctttacggc 5400
   acctcgaccc caaaaaactt gattagggtg atggttcacg tagtgggcca tcgccccgat 5460
   agacggtttt tcgccctttg acgctggagt tcacgttcct caatagtgga ctcttgttcc 5520
   aaactggaac aacactcaac cctatctcgg tctattcttt tgatttataa gggattttgc 5580
   cgatttcggc ctattggtta aaaaatgagc tgatttaaca aaaatttaac gcgaatttta 5640
   acaaaatatt aacgtttata atttcaggtg gcatctttcg gggaaatgtg cgcggaaccc 5700
   ctatttgttt atttttctaa atacattcaa atatgtatcc gctcatgaga caataaccct 5760
   gataaatgct tcaataatat tgaaaaagga agagtatgag tattcaacat ttccgtgtcg 5820
   cccttattcc cttttttgcg gcattttgcc ttcctgtttt tgctcaccca gaaacgctgg 5880
   tgaaagtaaa agatgctgaa gatcagttgg gtgcacgagt gggttacatc gaactggatc 5940
   tcaatagtgg taagatcctt gagagttttc gccccgaaga acgttttcca atgatgagca 6000
   cttttaaagt tctgctatgt ggcgcggtat tatcccgtat tgacgccggg caagagcaac      6060
   tcggtcgccg catacactat tctcagaatg acttggttga gtactcacca gtcacagaaa      6120
   agcatcttac ggatggcatg acagtaagag aattatgcag tgctgccata accatgagtg      6180
   ataacactgc ggccaactta cttctgacaa cgatcggagg accgaaggag ctaaccgctt      6240
   ttttgcacaa catgggggat catgtaactc gccttgatcg ttgggaaccg gagctgaatg      6300
   aagccatacc aaacgacgag cgtgacacca cgatgcctgt agtaatggta acaacgttgc      6360
   gcaaactatt aactggcgaa ctacttactc tagcttcccg gcaacaatta atagactgga      6420
   tggaggcgga taaagttgca ggaccacttc tgcgctcggc ccttccggct ggctggttta      6480
   ttgctgataa atctggagcc ggtgagcgtg ggtctcgcgg tatcattgca gcactggggc      6540
   cagatggtaa gccctcccgt atcgtagtta tctacacgac ggggagtcag gcaactatgg      6600
   atgaacgaaa tagacagatc gctgagatag gtgcctcact gattaagcat tggtaactgt      6660
   cagaccaagt ttactcatat atactttaga ttgatttaaa acttcatttt taatttaaaa      6720
   ggatctaggt gaagatcctt tttgataatc tcatgaccaa aatcccttaa cgtgagtttt      6780
   cgttccactg agcgtcagac cccgtagaaa agatcaaagg atcttcttga gatccttttt      6840
   ttctgcgcgt aatctgctgc ttgcaaacaa aaaaaccacc gctaccagcg gtggtttgtt      6900
   tgccggatca agagctacca actctttttc cgaaggtaac tggcttcagc agagcgcaga      6960
   taccaaatac tgtccttcta gtgtagccgt agttaggcca ccacttcaag aactctgtag      7020
   caccgcctac atacctcgct ctgctaatcc tgttaccagt ggctgctgcc agtggcgata      7080
   agtcgtgtct taccgggttg gactcaagac gatagttacc ggataaggcg cagcggtcgg      7140
   gctgaacggg gggttcgtgc acacagccca gcttggagcg aacgacctac accgaactga      7200
   gatacctaca gcgtgagcta tgagaaagcg ccacgcttcc cgaagggaga aaggcggaca      7260
   ggtatccggt aagcggcagg gtcggaacag gagagcgcac gagggagctt ccagggggaa      7320
   acgcctggta tctttatagt cctgtcgggt ttcgccacct ctgacttgag cgtcgatttt      7380
   tgtgatgctc gtcagggggg cggagcctat ggaaaaacgc cagcaacgcg gcctttttac      7440
   ggttcctggc cttttgctgc ggttttgctc acatgttctt tcctgcgtta tcccctgatt      7500
   ctgtggataa ccgtattacc gcctttgagt gagctgatac cgctcgccgc agccgaacga      7560
   ccgagcgcag cgagtcagtg agcgaggaag cggaag                                7596

## Beschreibung

### GEBIET DER ERFINDUNG

Die Erfindung liegt auf dem Gebiet der Gentherapie. Genauer beschäftigt sich die Erfindung mit der Bereitstellung einer Gentherapie für koronare Herzerkrankung und periphere Ischämie in Säugetieren.

### HINTERGRUND

Koronare Herzerkrankung ist in den Industrieländern, trotz einer verbesserten Therapie wie der Revaskularisierung eines verschlossenen Koronargefäßes, nach wie vor die häufigste Todesursache (Lloyd-Jones et al., Circulation 2010, 121:e46-e215). Neben der Manifestation der koronaren Herzerkrankung als akuter Herzinfarkt kann es auch durch den langsamen chronischen Verschluss eines Herzkranzgefäßes zu einer myokardialen Ischämie kommen, die im Verlauf zu einer Herzinsuffizienz bis hin zum Herzversagen führt (Suero et al., J Am Coll Cardiol 2001, 38:409-14).

Chronische ischämische Erkrankung von Herz- oder peripherem Muskel wird gegenwärtig durch chirurgische oder interventionelle Maßnahmen behandelt, um verengte oder okkludierte vaskuläre Netzwerke zu revaskularisieren. Auch wenn in den letzen Jahren die medikamentöse Therapie nach Wiedereröffnen des verschlossenen Gefäßes und somit das ereignisfreie Überleben der Pateinten deutlich verbessert werden konnte, entwickelt immer noch ein Teil der Patienten eine Herzinsuffizienz (Levy et al., N Engl J Med 2002, 347:1397-402). In einer wachsenden Patientenpopulation sind konventionelle therapeutische Strategien erschöpft und ein klinischer Nutzen wird von adjuvanten Neovaskularisationstherapien erwartet (Angiogenese/Arteriogenese).

Vorherige präklinische (Kupatt et al., J Am Coll Cardiol 2010, 56:414-22) und klinische Studien (Rissanen und Ylä-Herttuala, Mol Ther 2007, 15:1233-47) konnten keine Steigerung der Perfusion nachweisen, wenn Angiogenese (Kapillarwachstum) in Abwesenheit von Mikrogefäßreifung erzwungen wurde, d.h. Rekrutierung von Perizyten und glatten Muskelzellen (Jain, Nat Med 2003, 9:685-693*;* Potente et al., Cell 2011, 146:873-887). Außerdem verlängerte Angiogenese (kollaterales Wachstum), ein wesentliches Element von Durchflussverbesserung, nicht die Gehzeit in Patienten mit kritischer Gliedmaßenischämie, wenn unterstützende GM-CSF-Behandlung ohne Induktion von Mikrogefäßwachstum und - stabilisierung angewendet wurde (van Royen et al., Circulation 2005, 112:1040-6). Im Gegensatz dazu fand adaptive Kollateralisierung (Schierling et al., J Vasc Res 2009, 46:365-374*)* statt, wenn ein proangiogener Faktor wie VEGF-A mit den Reifungsfaktoren PDGF-B (Kupatt et al., J Am Coll Cardiol 2010, 56:414-22) oder Angiopoietin-1 (Smith et al., J Am Coll Cardiol 2012, 59:1320-8) kombiniert wurde. Auf der anderen Seite führte Hemmung von NF-κB-Signalgebung, die VEGF-A- und PDGF-B-Expression behindert, zu einem hyperverzweigten und unreifen kollateralen Netzwerk (Tirziu et al., Circulation 2012, 126:2589-600). Demnach ist ein Zuwachs an stabilen und regulierten Mikrogefäßen notwendig, um funktionale Neovaskularisation zu induzieren.

Das ereignisfreie Überleben der Patienten könnte durch eine Gentherapie zur Angiogenese, Arteriogenese und verbesserten Herzfunktion deutlich verbessert werden. Hierfür ist es jedoch notwendig, den richtigen Gentherapie-Vektor und die richtigen Zielmoleküle zu verwenden. Die vorliegende Erfindung löst dieses Problem durch Bereitstellung von AAV-Vektoren zur vaskulären Gentherapie in koronarer Herzerkrankung.

Hinkel et al. (Cardiovasc. Res. 2012, 93(S1):S117) offenbart für Thymosin B4 kodierende AAV-Vektoren zur Behandlung von ischämischer Kardiomyopathie.

### ZUSAMMENFASSUNG

In einer Ausführungsform betrifft die Erfindung einen Adeno-assoziierten viralen Vektor (AAV-Vektor) umfassend ein Gen, das für einen Myocardin-verwandten Transkriptionsfaktor A (MRTF-A) kodiert.

Der AAV-Vektor kann ein AAV2/9 oder ein mit Hüllproteinen aus AAV9 pseudotypisierter AAV-Vektor sein, vorzugsweise AAV2.9, AAV1.9 oder AAV6.9.

In einer Ausführungsform steht das Gen unter der Kontrolle eines kardiospezifischen Promotors. In einer Ausführungsform steht das Gen unter der Kontrolle eines CMV-Promotors, eines MRC2-Promotors, eines MyoD-Promotors oder eines Troponin-Promotors.

Ferner betrifft die Erfindung auch eine pharmazeutische Zusammensetzung umfassend einen erfindungsgemäßen AAV-Vektor und einen pharmazeutisch akzeptablen Träger.

Die Erfindung umfasst auch einen erfindungsgemäßen AAV-Vektor oder eine erfindungsgemäße pharmazeutische Zusammensetzung zur Verwendung als Medikament. In einer Ausführungsform dient der erfindungsgemäße AAV-Vektor oder die erfindungsgemäße pharmazeutische Zusammensetzung zur Verwendung in der Behandlung von koronarer Herzerkrankung oder peripherer Ischämie in einem Säugetier, vorzugsweise in einem Menschen, einer Maus, einem Kaninchen oder einem Schwein. Die koronare Herzerkrankung kann akuter Herzinfarkt, myokardiale Ischämie stabile Angina pectoris und/oder hibernierendes Myokard sein.

In einer Ausführungsform ist das Säugetier ein menschlicher No-Option-Patient.

### BESCHREIBUNG DER FIGUREN

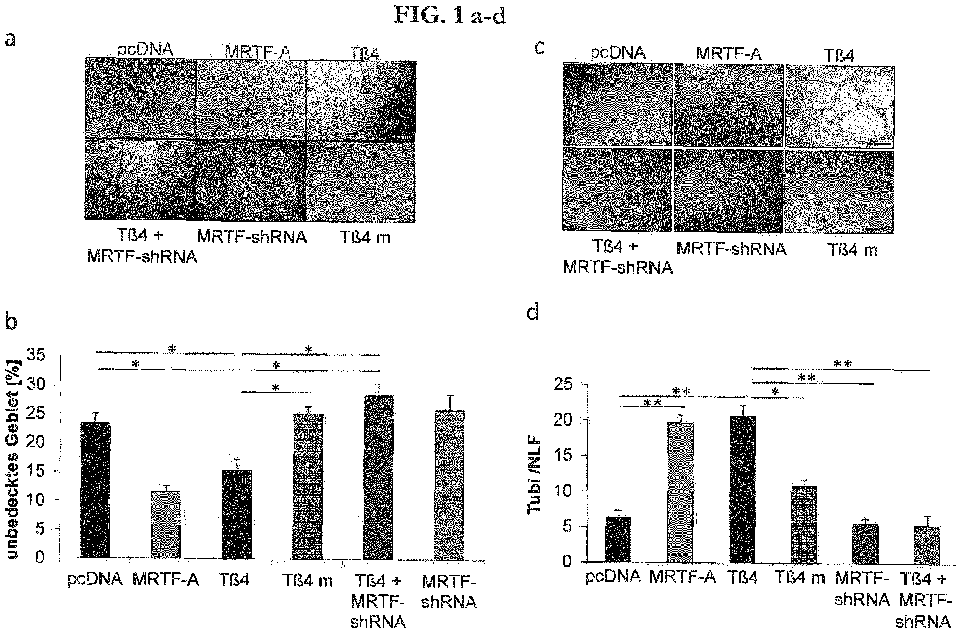

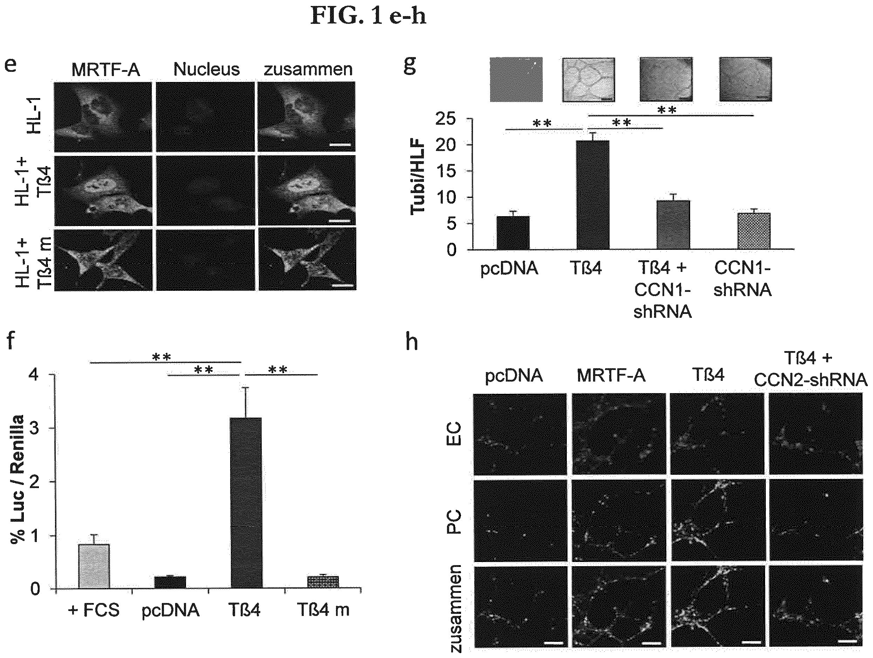

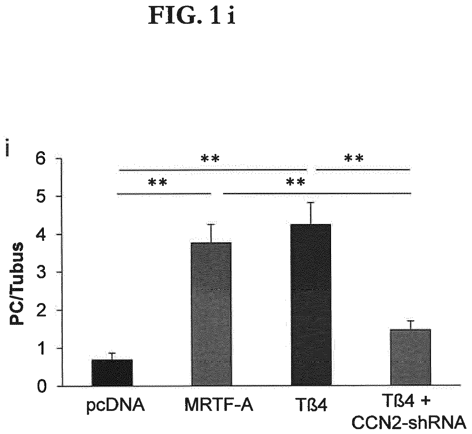

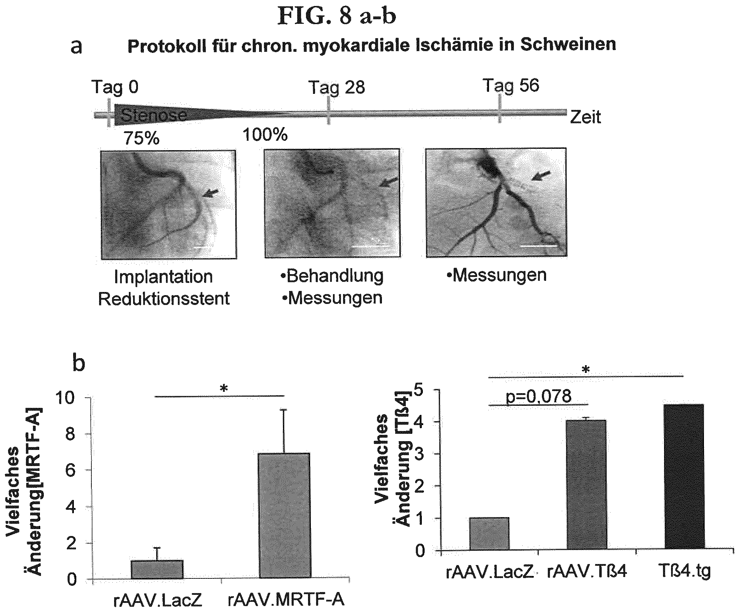

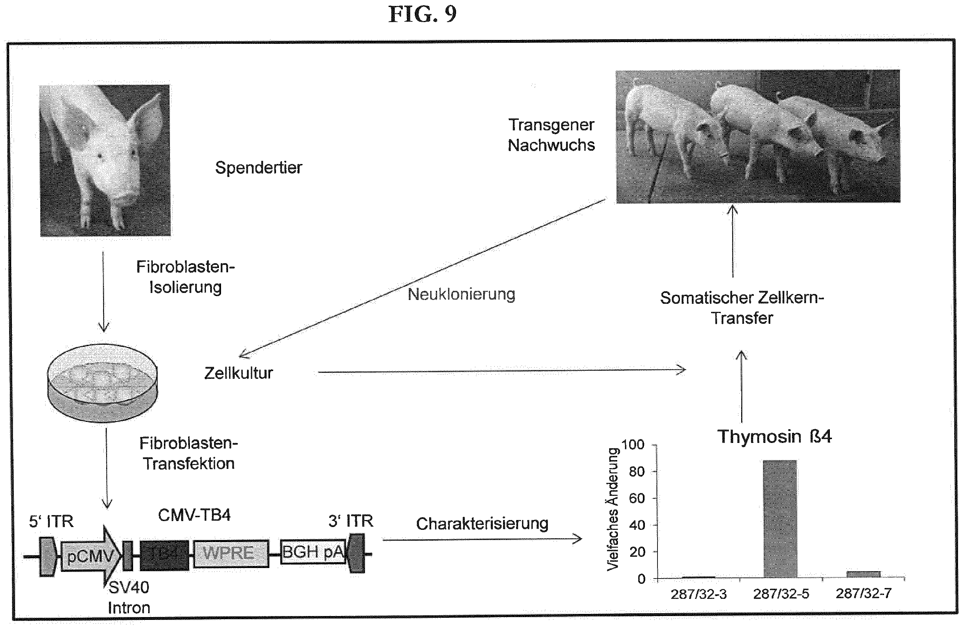

FIG.1: Durch MRTF-Aktivierung und Translokation in den Zellkern induzierte Angiogenese über CCN1- und CCN2-Aktivierung
   **(a,b)** MRTF-A-Transfektion erhöhte Endothelzellenmigration in einem Wundkratz-Test in vitro (umrandete Fläche = unbedeckte Fläche); und **(c,d)** Tubusbildung von humanen mikrovaskulären Endothelzellen (HMECs) in vitro (lpf = Niedrigleistungsfeld). Überexpression von Tβ4 zeigte ähnliche Effekte, falls keine MRTF-shRNA mitverabreicht wurde oder eine Tβ4-Mutante (Tß4m) verwendet wurde, welcher das G-Actin-Bindemotiv KLKKTET fehlt (Größenbalken: 200 µm). **(e)** Tβ4-Transfektion von myozytischen HL-1-Zellen ermöglichte Translokation in den Zellkern (blaue Fluoreszenz) von MRTF-A (grüne Fluoreszenz), ein Effekt der fehlte, wenn das Tβ4m-Konstrukt ohne die G-Actin-Bindungsstelle verwendet wurde (Größenbalken: 20 µm). **(f)** Tβ4-Transfektion von HL-1-Zellen induzierte einen MRTF-SRF-sensitiven Luicferase-Reporter (umfassend drei Kopien der SRF-Bindungsstelle c-fos = p3DA.Luc, vgl. Posern et al., Mol. Biol. Cell 2002, 13:4167-78), im Gegensatz zu Transfektion mit Tβ4-Mutante. **(g)** Tβ4-induzierte Tubusbildung wurde im Fall von shRNA-Kotransfektion des MRTF-SRF-Zielgens CCN1 (Cyr61) beseitigt (Größenbalken: 200 µm). **(h,i)** Tubusreifung, ausgewertet als Perizytenrekrutierung (PC, grüne Fluoreszenz) an endotheliale Ringe (EC-Ringe, rote Fluoreszenz; Größenbalken: 200 µm), wurde durch MRTF-A und Tβ4 induziert. Kotransfektion von shRNA gegen das MRTF-Zielgen CCN2 (CTGF) beseitigte den Tβ4-Effekt (Durchschnitt ± Stabw., n=5, * p<0,05, ** p<0,001).

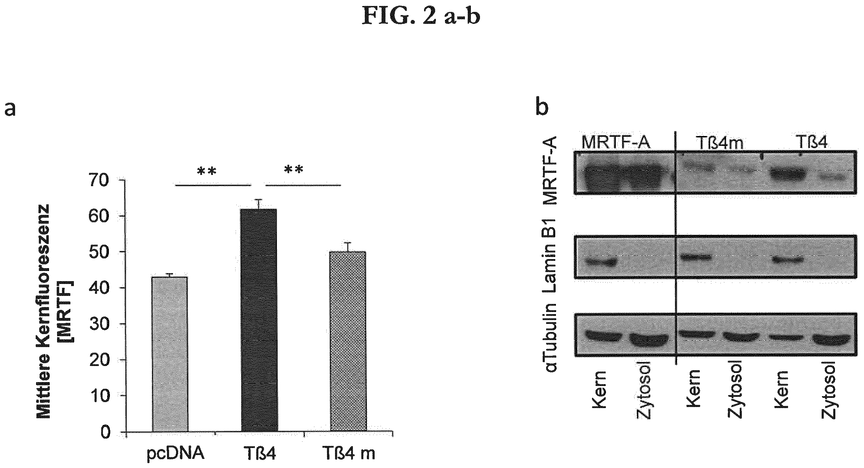

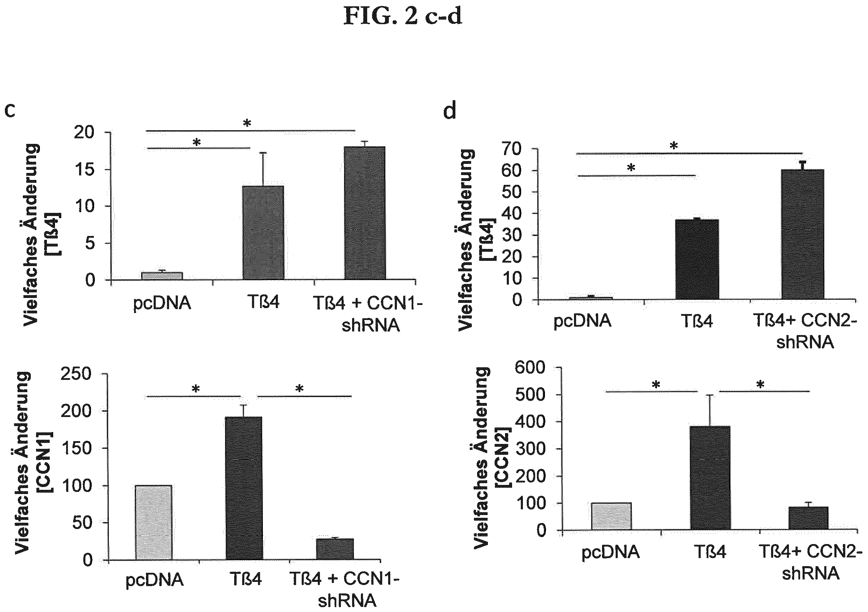

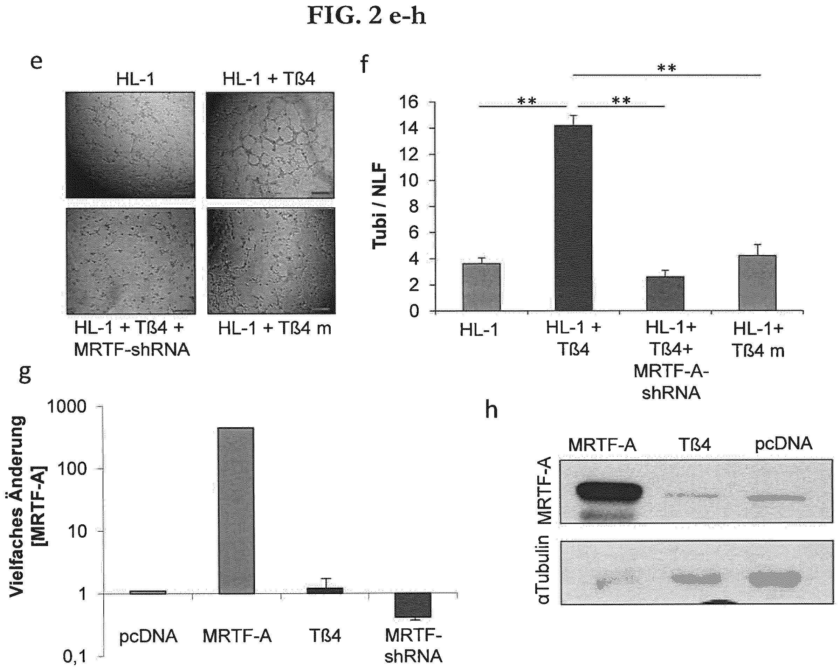

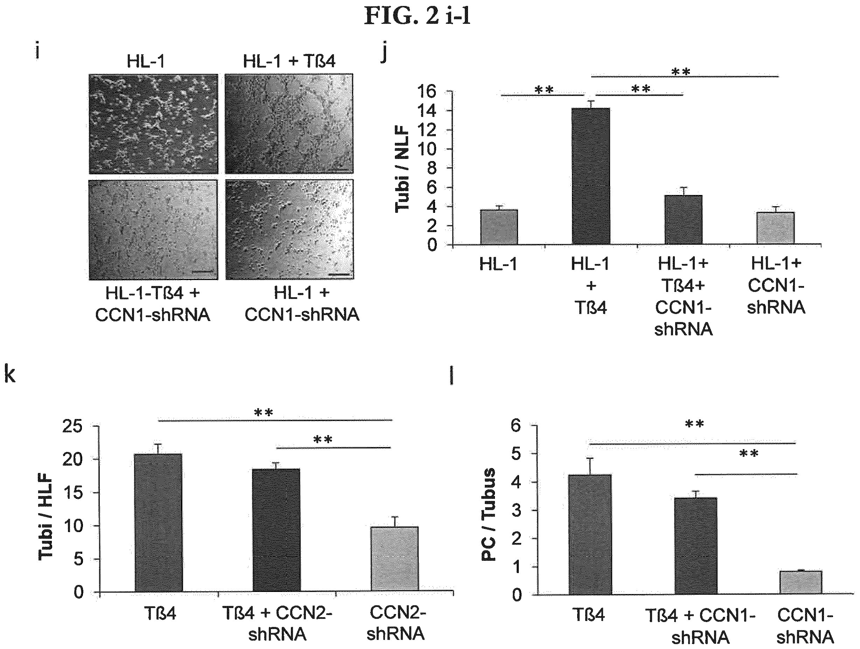

FIG. 2: Die Tβ4-MRTF-A-Signalkaskade induziert Angiogenese in vitro
   **(a)** Tβ4-Transfektion von kardiomyozytischen HL-1-Zellen ermöglicht die Translokation von MRTF in den Zellkern, ein Effekt, der fehlt, wenn ein Tβ4m-Konstrukt ohne Actin-Bindungsstelle verwendet wurde. **(b)** Analyse des MRTF-A-Proteinspiegels im Zellkern mittels Western Blot zeigte einen erhöhten MRTF-A-Proteinspiegel nach Tβ4-Überexpression in HL-1-Zellen. Tβ4m erhöhte den MRTF-A-Spiegel nicht. **(c,d)** qRT-PCR zeigt, dass CCN1/2-shRNA die Anreicherung von CCNl/2-Transkripten nach Tβ4-Expression verhinderte. **(e,f)** rAAV.Tβ4-transduzierte kardiomyozytische HL-1-Zellen induzierten Angiogenese (Tubusbildung) in mit HL-1-Zellen kokultivierten Endothelzellen, wenn keine MRTF-shRNA kotransduziert wurde, wohingegen rAAV.Tβ4m keinen Effekt hatte (Größenbalken: 200 µm). **(g,h)** MRTF-A-mRNA-Expressionsniveaus (g) sowie MRTF-A-Proteinspiegel (h) wurden durch Tβ4-Überexpression nicht beeinflusst, aber nach MRTF-A-Transfektion signifikant erhöht. **(i,j)** Tubusbildung nach Tβ4-Freisetzung aus rAAV.Tβ4-transduzierten HL-1-Zellen wurde durch CCN1-shRNA gestört (Größenbalken: 200 µm). **(k,l)** Kotransfektion von rAAV.Tβ4 und CCN2-shRNA beeinflusste die Tβ4-induzierte Tubusbildung nicht. Des Weiteren veränderte CCN1-shRNA nicht die Tβ4-induzierte Rekrutierung von Perizyten-artigen Zellen (Durchschnitt ± Stabw., n=5, * p<0,05, ** p<0,001).

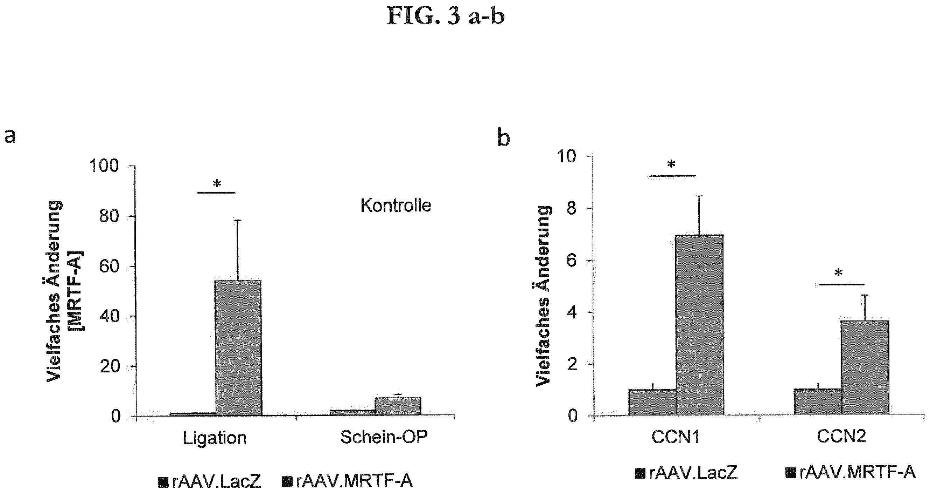

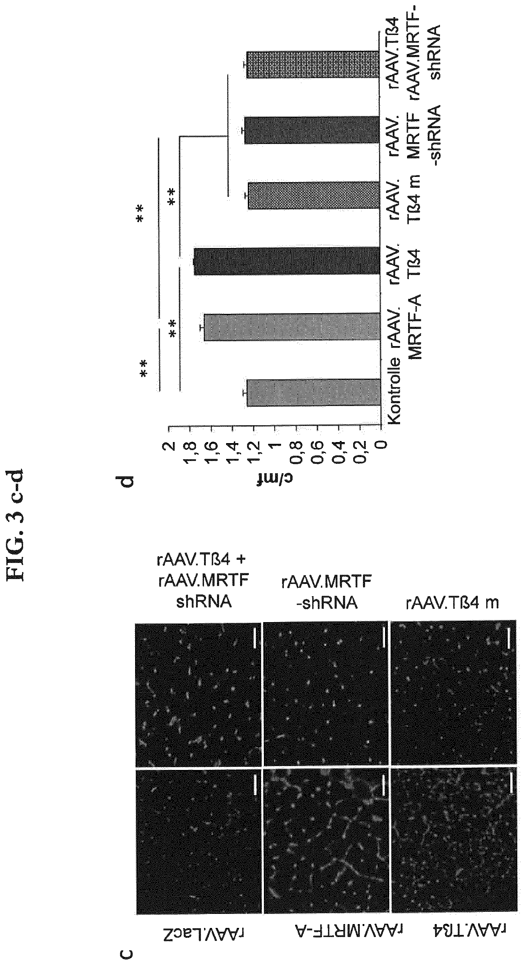

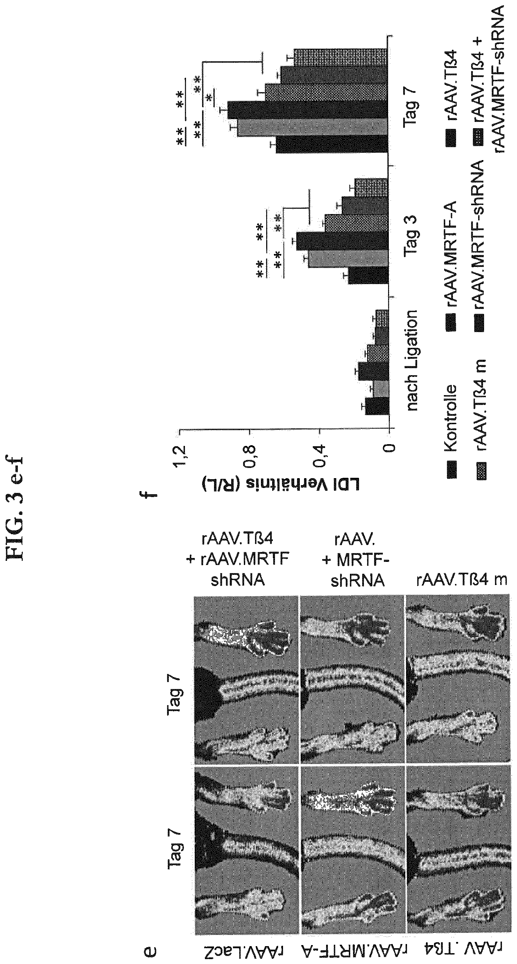

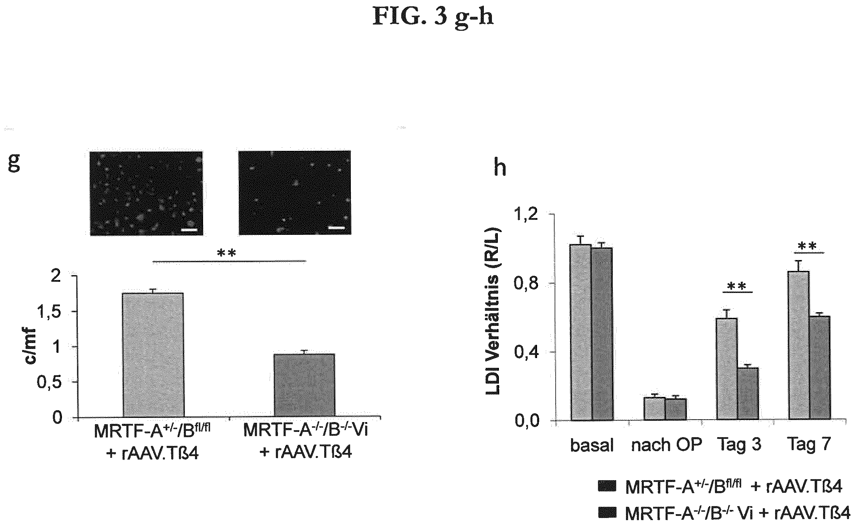

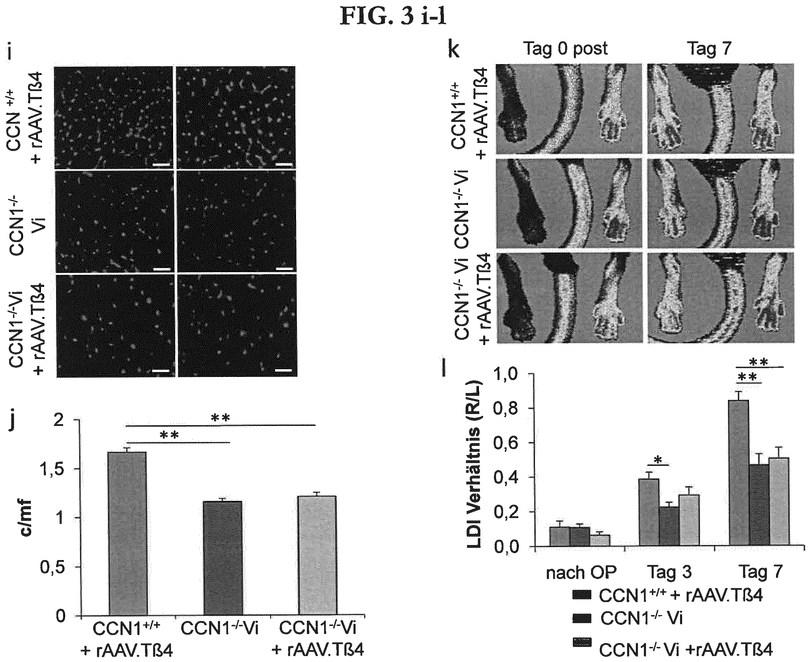

FIG.3: Bedeutung von MRTF-Signalgebung für Neovaskularisation in vivo
   **(a)** qRT-PCR-Analyse zeigte eine Erhöhung von MRTF-A in dem rAAV.MRTF-A-transduzierten ischämischen Hinterbein. **(b)** rAAV.MRTF-A induzierte MRTF/SRF-Zielgene CCN1 und CCN2 in vivo. **(c,d)** rAAV.MRTF-A-Transduktion erhöhte das Kapillaren-Muskelfaser-Verhältnis (c/mf), ähnlich wie der MRTF-Aktivator Tβ4. rAAV.Tβ4m, eine Mutante ohne die G-Actin-Bindedomäne, oder Koapplikation von Tβ4 und rAAV.MRTF-shRNA, hatten keinen Effekt (PECAM-1-Färbung, Größenbalken: 100 µm). **(e,f)** Funktional verbesserte Transduktion mit rAAV.MRTF-A und -Tβ4, aber nicht rAAV.Tβ4m oder rAAV.Tβ4+MRTF-shRNA die Perfusion des Hinterbeins an Tag 3 und Tag 7. **(g)** Nach rAAV.Cre-Vektor-induzierter MRTF-B-Deletion in MRTF-A-defizienten Mäusen (Mrtfa^{-/-} /b^{flox/flox}+rAAV.Cre = MRTF-A/B^{-/-}Vi) konnte Tβ4-Transduktion keine Angiogenese induzieren, im Gegensatz zu Mrtfa^{+/-}/b^{flox/flox}-Mäusen (= MRTF-A/B^{+/-}). **(h)** Mit rAAV.Tβ4 gesteigerte Perfusion wurde in MRTF-A/B^{-/-}Vi-Mäusen unterdrückt. **(i,j)** In CCN1^{-/-}Vi-Mäusen (= CCN1^{flox/flox}+rAAV.Cre) wurde der Anstieg im Kapillaren-Muskelfaser-Verhältnis unterdrückt (PECAM-1-Färbung, Größenbalken: 100 µm), wie auch der Zuwachs an Hinterbein-Perfusion **(k,l)** (Durchschnitt ± Stabw., n=5, * p<0,05, ** p<0,001).

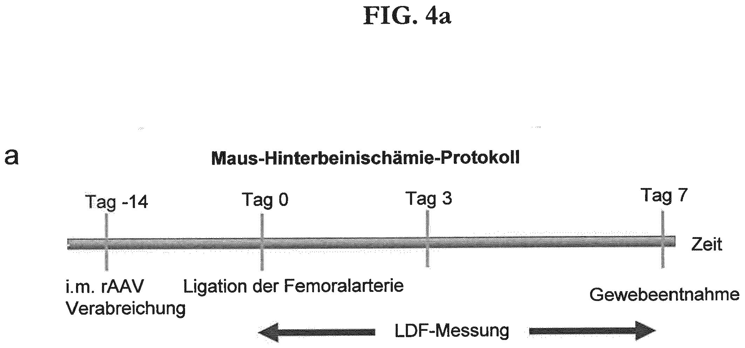

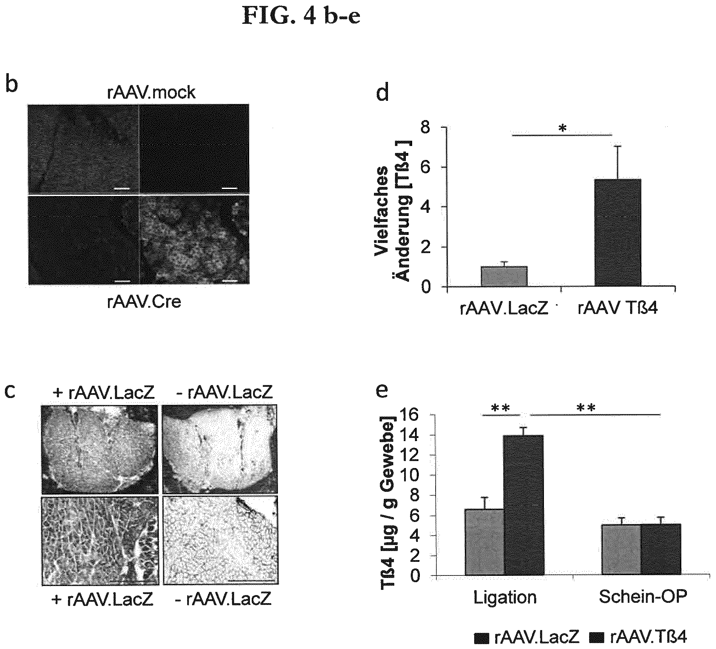

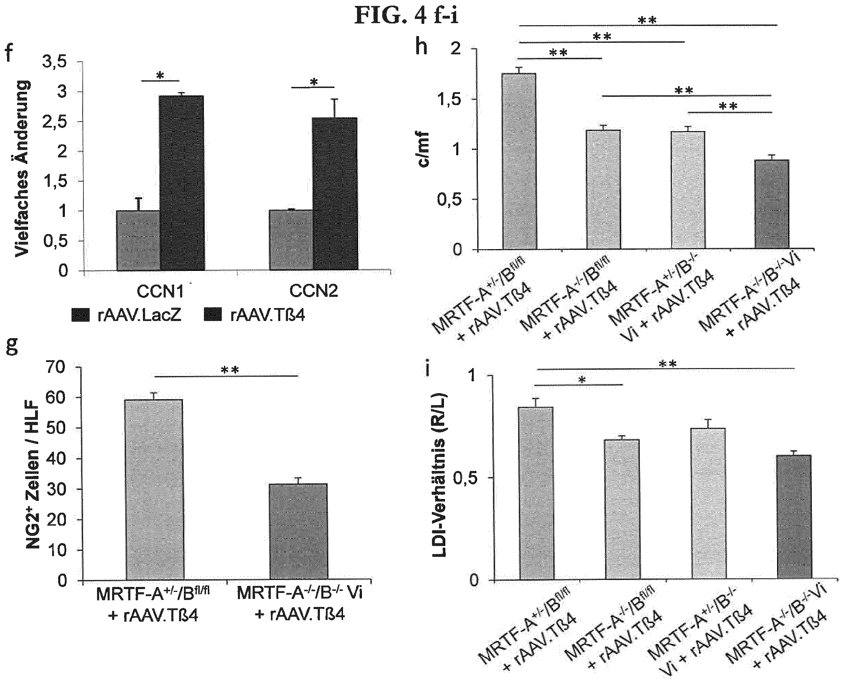

FIG. 4: MRTF-A-induziertes Gefäßwachstum in Hinterbeinischämie der Maus
   **(a)** Protokoll zur Hinterbeinischämie in der Maus. Intramuskuläre (i.m.) rAAV-Verabreichung wurde an Tag -14 durchgeführt und die Femoralarterie wurde an Tag 0 ligiert. Nachfolgende Laser-Doppler-Durchflussmessungen (LDF) wurden an Tagen 0, 3 und 7 durchgeführt, **(b)** I.m. Injektion von rAAV.Cre induzierte homogene Muskeltransduktion, angezeigt durch einen Wechsel von Tomato-Fluoreszenz (rot) zu GFP-Fluoreszenz (grün) in Tomato-Reportergen-Mäusen. **(c)** I.m. Injektion von rAAV.LacZ (3x10¹² Viruspartikel) führte zu einer homogenen Transduktion (blaue Färbung) des anvisierten Hinterbeins, aber nicht des gegenseitigen. **(d)** qRT-PCR-Nachweis von Tβ4 in den rAAV.Tβ4-transduzierten ischämischen Hinterbeinen, aber nicht in rAAV.LacZ-transduzierten Hinterbeinen. **(e)** HPLC-Analyse zeigte einen Anstieg der Tβ4-Proteinkonzentration in den rAAV.Tβ4-transduzierten ischämischen Hinterbeinen. **(f)** rAAV.Tβ4 induzierte MRTF-Zielgene CCN1 und CCN2 in vivo. **(g)** Tβ4-induzierte Reifung der Kapillaren (Perizyten-Auskleidung, NG2-Färbung) war in MRTF-A/B^{-/-}Vi-Hinterbeinen unterdrückt. **(h,i)** Sowohl in MRTF-A^{-/-}/B^{flox/flox}-Mäusen (MRTF-A-Knockout) als auch MRTF-A^{+/-}/B^{-/-}Vi-Mäusen (MRTF-B-Knockout) verursachte rAAV.Tβ4-Transduktion eine Erhöhung der Kapillarendichte (h) und Perfusion (i). Jedoch war der rAAV.Tβ4-Effekt in Wildtyp-Mäusen (MRTF-A^{+/-}/B^{flox/flox}) am höchsten. Außerdem gab es keinen signifikanten Unterschied zwischen MRTF-A- und MRTF-B-Knockout-Mäusen (Durchschnitt ± Stabw., n=4, * p<0,05 vs. Kontrolle).

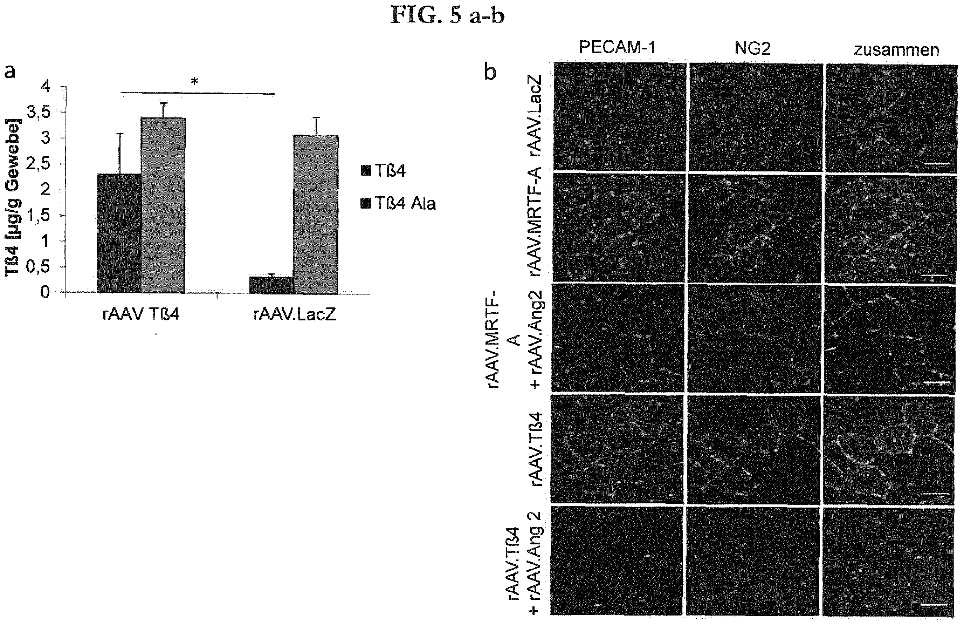

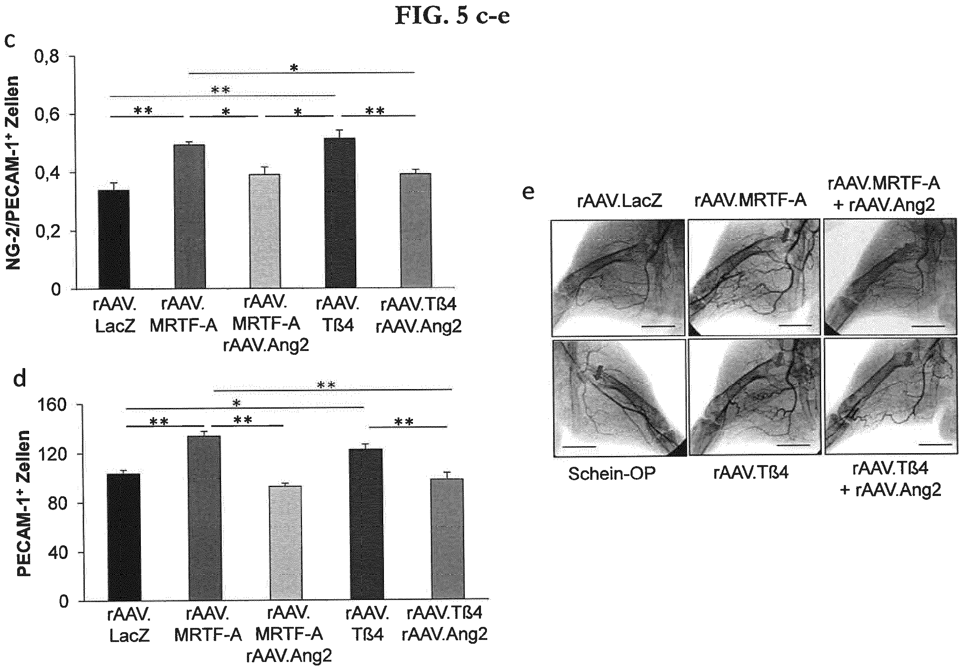

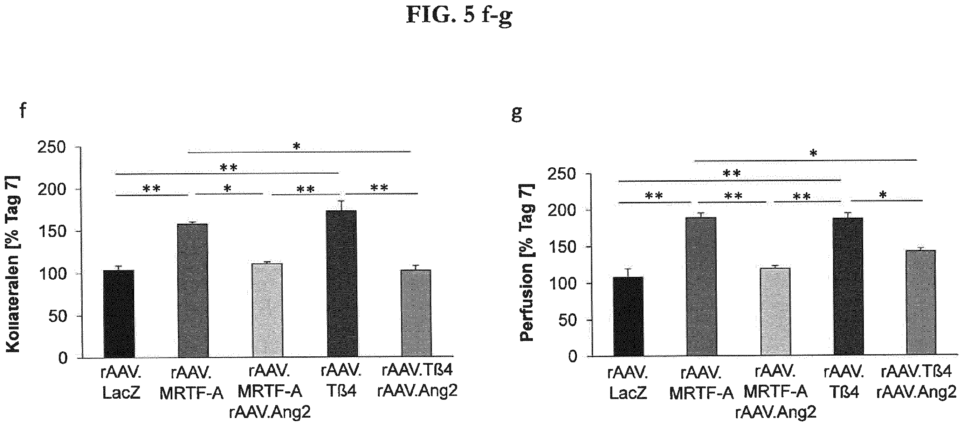

FIG. 5: Tβ4/MRTF-A-induzierte Mikrogefäßreifung: essentielle Rolle für Kollateralenwachstum und verbesserte Perfusion
   **(a)** HPLC-Analyse zeigte einen signifikanten Anstieg von Tβ4-Protein nach rAAV.Tβ4-Transduktion von ischämischen Kaninchenhinterbeinen, wohingegen kaninchenspezifisches Tβ4-Ala unverändert blieb. **(b-d)** rAAV.MRTF-A- oder rAAV.Tβ4-Verabreichung erhöhte die Kapillardichte (PECAM-1-Färbung) sowie die Perizytenauskleidung (NG2-Färbung, Größenbalken: 50 µm), die beide durch Koapplikation von Angiopoietin 2 (rAAV.Ang2) beseitigt wurden. **(e,f)** Angiographien von ischämischen Hinterbeinen an Tag 35 zeigten eine erhöhte Kollateralenbildung in rAAV.MRTF-A- und rAAV.Tβ4-behandelten Tieren (Pfeile zeigen die Stelle der Exzision der Femoralarterie an). Koapplikation von rAAV.Ang2 beseitigte diesen Effekt, **(g)** rAAV.MRTF-A und rAAV.Tβ4 induzierten einen Zuwachs an Perfusion in ischämischen Hinterbeinen, sofern nicht rAAV.Ang2 oder L-NAME, das die Stickoxidbildung hemmt, koappliziert wurden (Durchschnitt ± Stabw., n=5, * p<0,05, ** p<0,001).

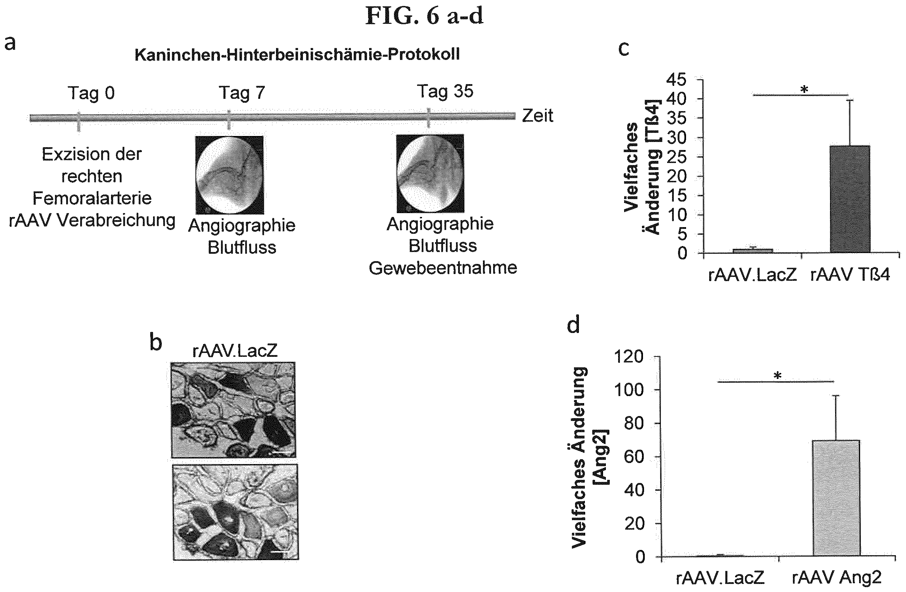

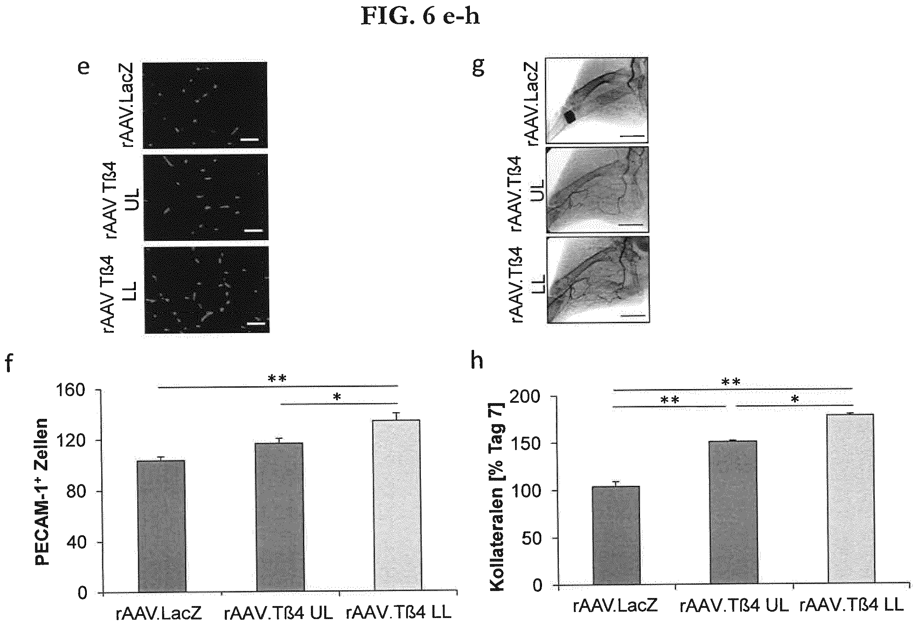

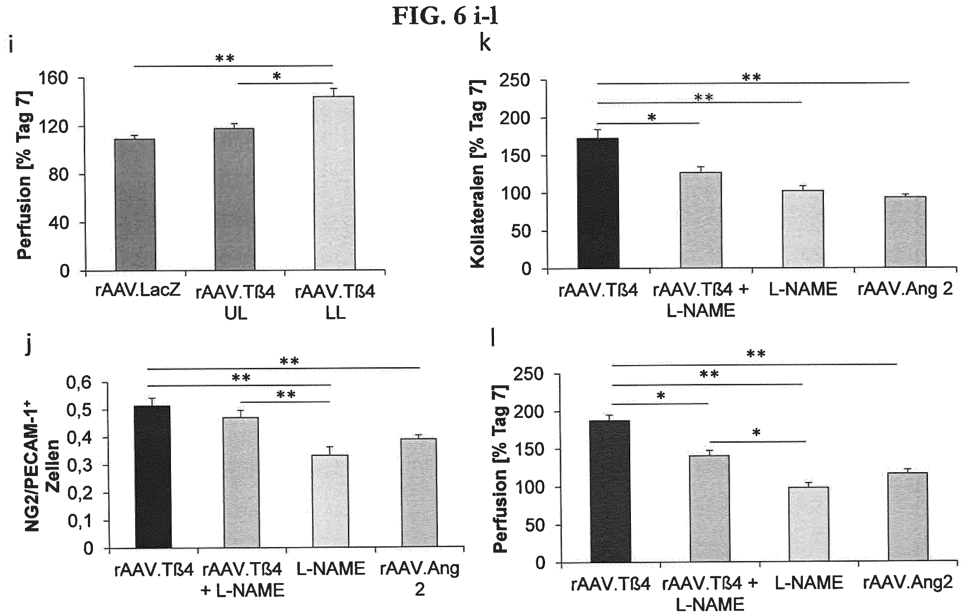

FIG. 6: Tβ4-MRTF-A-induziertes Gefäßwachstum in Kaninchen
   **(a)** Protokoll eines Modells für Kaninchen-Hinterbeinischämie (Femoralarterien-Exzision). **(b)** β-Galactosidase-Färbung 5 Wochen nach i.m. Injektion von rAAV.LacZ in das Kaninchenhinterbein. **(c,d)** qRT-PCR von Tβ4 (c) und Angiopoietin 2 (d), normalisiert zu GAPDH, in Kontroll- und Behandlungstieren (n=3). **(e,f)** Tβ4-Überexpression nur im Unterschenkel (rAAV.Tβ4 LL) erhöhte die Kapillarendichte (PECAM-1-Färbung) im Unterschenkel, wohingegen Tβ4-Transduktion nur im Oberschenkel (rAAV.Tβ4 UL) die Kapillarisierung im Unterschenkel nicht beeinflusste (Größenbalken: 50 µm). **(g,h)** Kollateralisierung erhöhte sich in der rAAV.Tβ4 UL- und zu einem sogar noch größeren Ausmaß in der rAAV.Tβ4 LL-Gruppe, wohingegen sich Perfusion **(i)** nur in der rAAV.Tβ4 LL- und nicht in der rAAV.Tβ4 UL-Gruppe erhöhte. **(j)** Verglichen mit rAAV.Tβ4-transduzierten Kaninchen verringerte die Mitverabreichung von L-NAME das NG2/PECAM-1-Verhältnis nicht, was ein robustes und ausgeglichenes Mikrogefäßwachstum anzeigte. Im Gegensatz dazu war L-NAME- oder rAAV.Ang2-Behandlung allein nicht in der Lage, das NG2-PECAM-1-Verhältnis so stark zu erhöhen wie rAAV.Tβ4. **(k,l)** Tβ4-abhängiger Anstieg der Kollateralisierung und Perfusion war signifikant reduziert, wenn L-NAME mitverabreicht wurde (Durchschnitt ± Stabw., n=5, ** p<0,01).

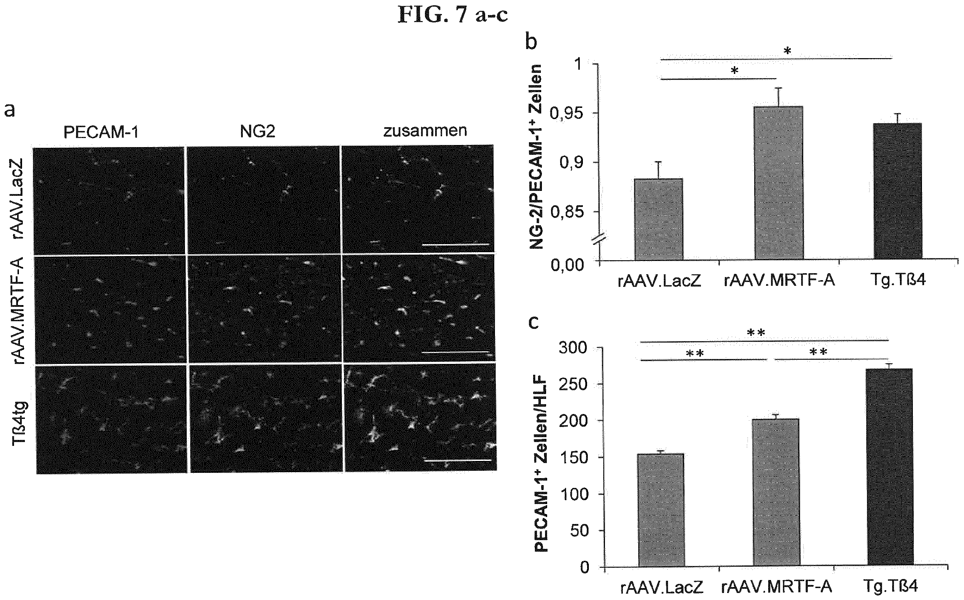

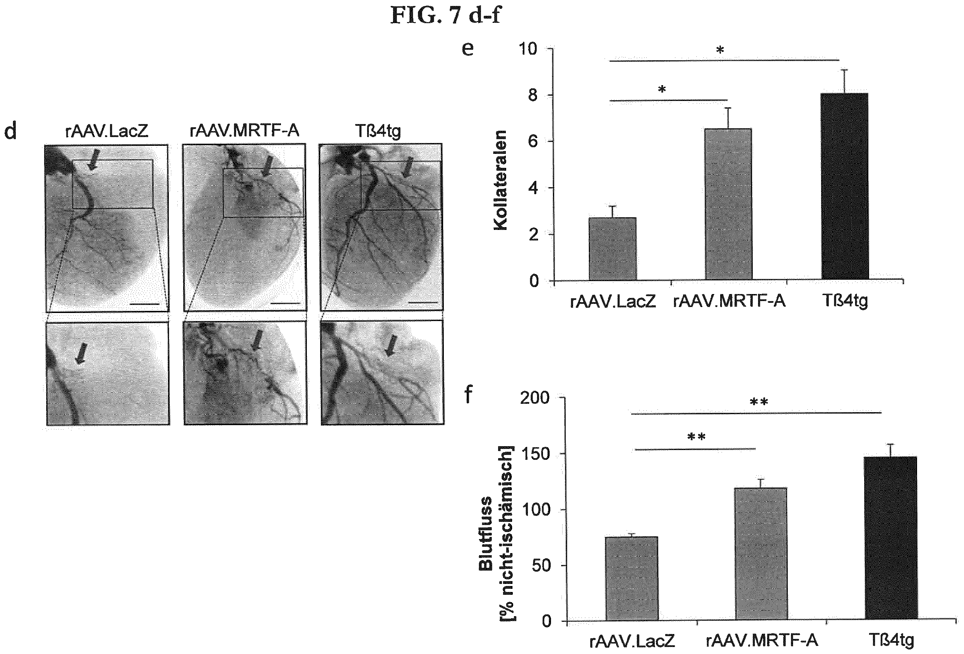

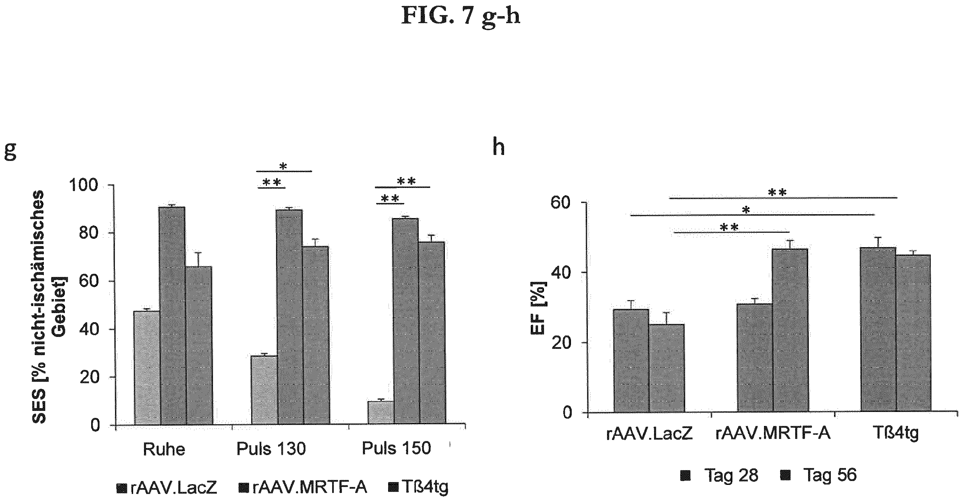

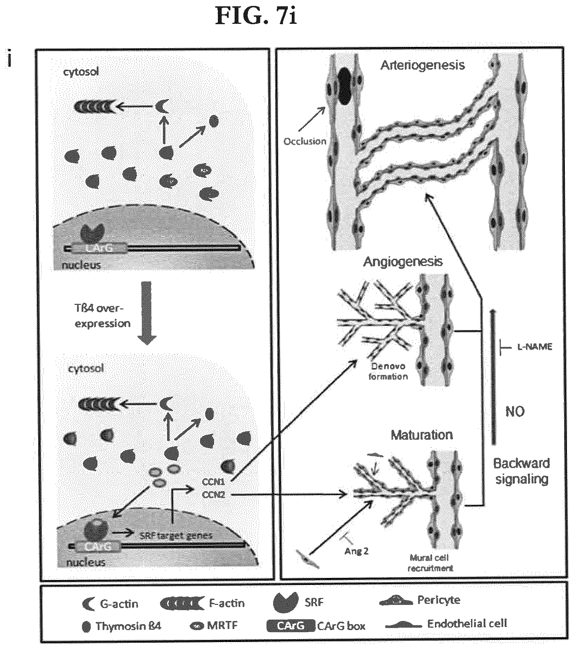

FIG. 7: MRTF-A verbessert Kollateralenbildung und Perfusion in hibernierendem Myokard von Schweinen
   **(a-c)** In hibernierendem Schweinemyokard (vgl. Fig. 8a) induzierten rAAV.MRTF-A-Transduktion sowie ubiquitäre Überexpression von Tβ4 (Tβ4tg, vgl. Fig. 9) Kapillarsprossung (PECAM-1-Färbung, Größenbalken: 50 µm) und Perizyten-Auskleidung (NG2-Färbung). **(d,e)** Des Weiteren wurde Kollateralenwachstum in rAAV.MRTF-A-transduzierten Herzen nachgewiesen, ähnlich zu Tβ4tg-Herzen. **(f)** Die regionale Flussreserve, erhalten durch schnelle Vorhofstimulation (130 Schläge pro Minute), war in rAAV-MRTF-A-transduzierten und Tβ4-transgenen Herzen erhöht. **(g)** Regionale Myokardfunktion, gemessen durch subendokardiale Segmentverkürzung in Ruhe und unter Vorhofstimulation (130 und 150 Schläge pro Minute), zeigte eine verbesserte funktionelle Reserve entweder durch rAAV.MRTF-A-Transduktion oder in Tβ4tg-Herzen. **(h)** Die Auswurffraktion, ein Parameter von globaler Myokardfunktion, verbesserte sich in rAAV.MRTF-A-transduzierten Tieren an Tag 56 verglichen mit Tag 28. Konstitutiv überexprimierende Tiere (Tβ4tg) hingegen zeigten keinen Funktionsverlust an Tag 28. **(i)** Mechanismen MRTF-vermittelter therapeutischer Neovaskularisation: MRTF-A- oder Tβ4-Transduktion induziert eine erhöhte Menge von an G-Actin nicht gebundenem MRTF-A, das nach Translokation in den Zellkern mit SRF wechselwirkt und z.B. CCN1 und CCN2 als Zielgene induziert. CCN1 ermöglich Kapillarwachstum (Angiogenese), während CCN2 die Perizyten-Auskleidung erhöht (Gefäßreifung). Zusammen induzieren diese Mechanismen Kollateralenwachstum in einer Stickoxid-abhängigen Weise, was zu therapeutischer Neovaskularisation führt (Durchschnitt ± Stabw., n=5, * p<0,05, ** p<0,001).

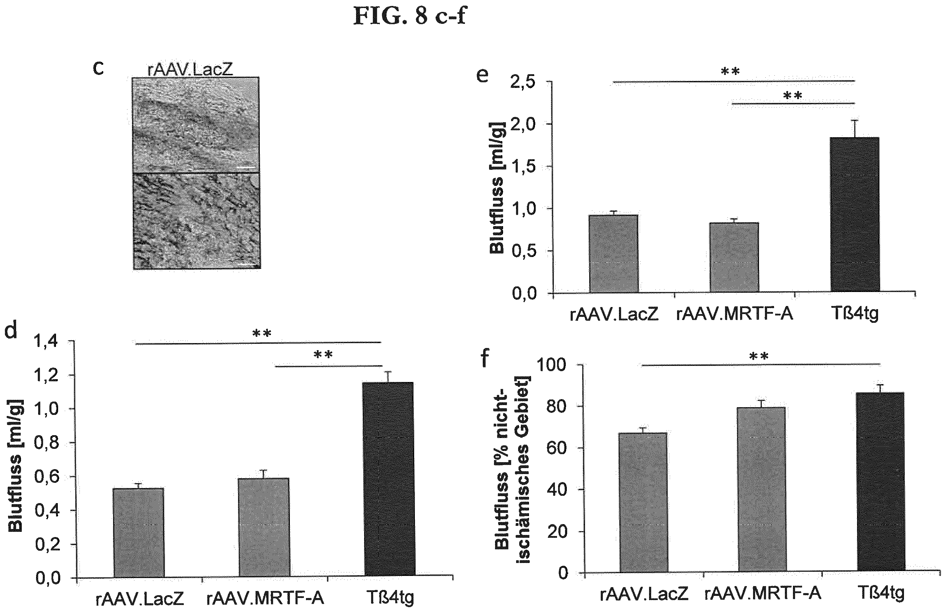

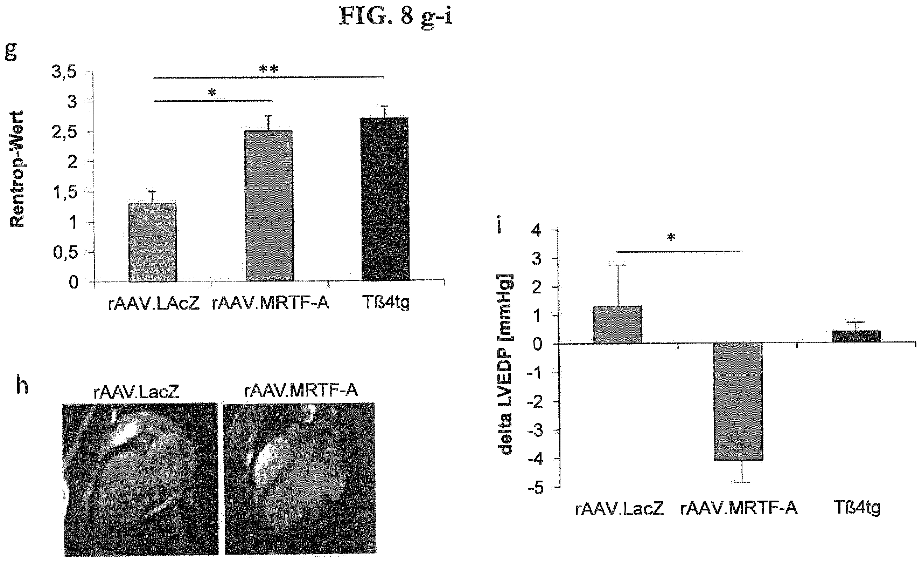

FIG. 8: Funktionale Effizienz der Tβ4-MRTF-A-Achse in chronisch ischämischen Schweineherzen
   **(a)** Protokoll des Schweinemodells für hibernierendes Myokard. **(b)** RT-PCR-Nachweis von MRTF-A und Tβ4 in Kontrollschweinen im Vergleich zu rAAV.Tβ4- und Tβ4-transgenen (Tβ4tg) Schweineherzen, **(c)** Beispiele von LacZ-Färbung (blau) nach rAAV.LacZ-Retroinfusion (5x10¹² Viruspartikel) ins Schweineherz, **(d)** Vor der Behandlung (an Tag 28) zeigte die Retentionsanalyse von fluoreszierenden Mikrokugeln in Ruhe einen verringerten Blutfluss im ischämischen Gebiet von rAAV.LacZ- und rAAV.MRTF-A-Herzen, aber nicht in Tβ4tg-Herzen, ähnlich zur Flussreserve **(e)** bei schneller Herzfrequenz (130 bpm). **(f)** 4 Wochen nach Behandlung (Tag 56) verbesserte sich der regionale myokardiale Blutfluss in Ruhe in rAAV.MRTF-A- und Tβ4tg-Tieren. **(g)** Außerdem zeigte der Rentrop-Wert eine erhöhte Kollateralisation an Tag 56 in rAAV.MRTF-A-transduzierten oder Tβ4tg-Herzen. **(h)** Beispiele für MRT-Analyse an Tag 56 für Kontroll- (links) und rAAV.MRTF-A-behandelte Schweineherzen, **(i)** Der enddiastolische Druck in der linken Herzkammer (LVEDP) stieg in ischämischen Herzen von Tag 28 zu Tag 56 an, wenn MRTF-A nicht überexprimiert wurde. Tβ4tg-Herzen, die Tβ4 konstitutiv überexprimierten, zeigten keine Veränderung von Tag 28 zu Tag 56 (Durchschnitt ± Stabw., n=5, * p<0,05, ** p<0,001).
FIG. 9: Herstellung von Tβ4-transgenen Schweinen
   Fibroblasten aus Spenderschweinen wurden isoliert und kultiviert. pCMV-Tβ4 wurde mittels Elektroporation transfiziert und die Zellen wurden über 14 Tage kultiviert. Nach Detektion von stabiler Transfektion von Tβ4 wurde ein somatischer Zellkerntransfer in Schweineeizellen durchgeführt. Nachkommen wurden auf Tβ4-Expression analysiert und Fibroblasten von Tβ4-exprimierenden Tieren wurden kultiviert und anschließend für einen zweiten somatischen Zellkerntransfer verwendet. Nach Genotypisierung wurden Tiere dieser Generation für das Schweinemodell von chronischer Ischämie verwendet.

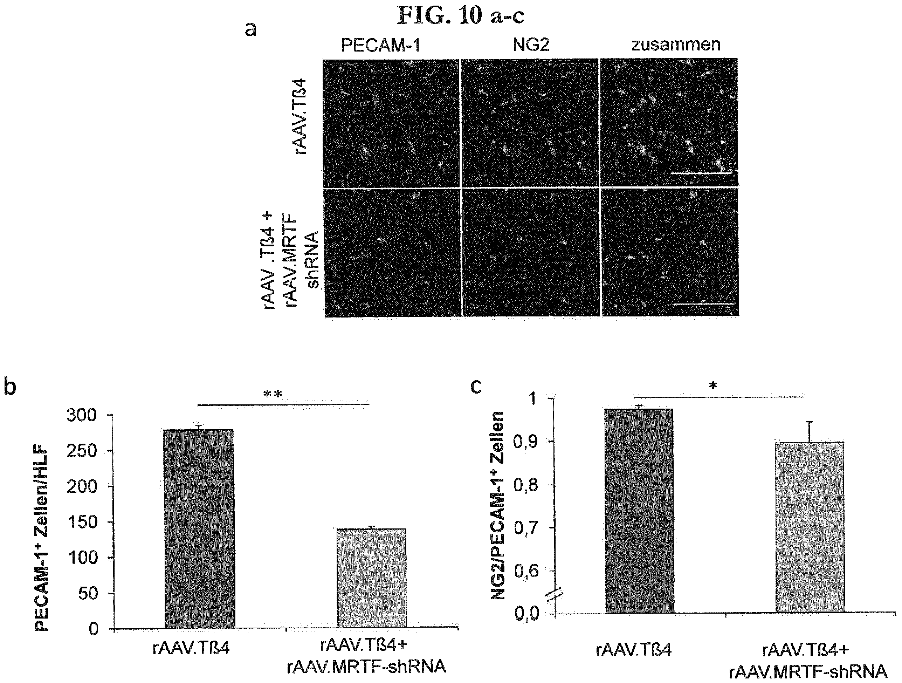

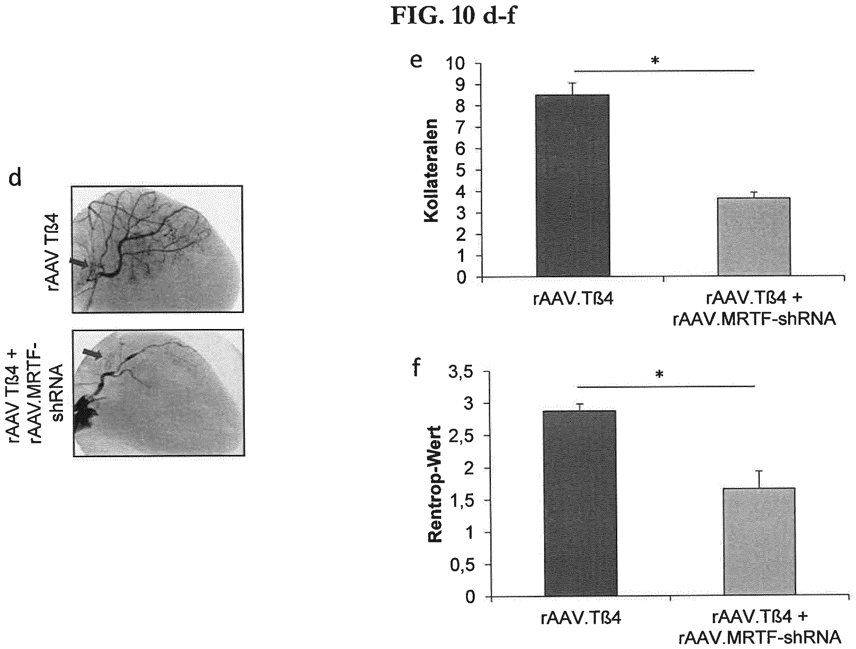

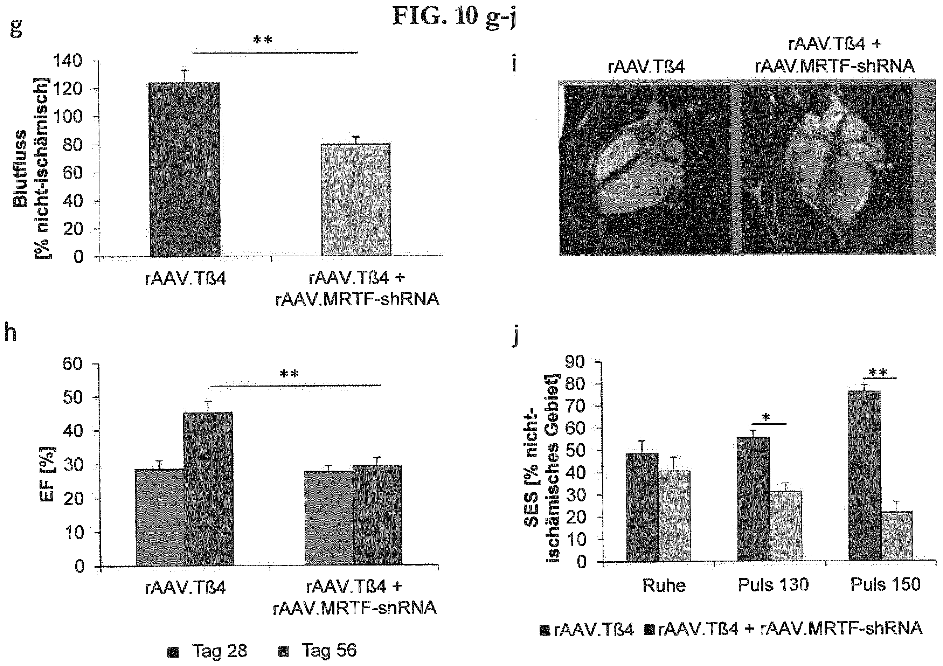

FIG. 10: MRTFs sind erforderlich für Tβ4-induzierte Kardioprotektion
   **(a,b)** rAAV.Tβ4 induzierte Kapillarwachstum (PECAM-1-Färbung) und **(c)** Perizyten-Auskleidung (NG2-Färbung, Größenbalken 50 µm), falls nicht Mitverabreichung von rAAV.MRTF-shRNA beide Prozesse verhinderte. **(d,e)** Kollateralenwachstum wurde in rAAV.Tβ4-transduzierten Tieren nachgewiesen, aber nicht nach Mitverabreichung von rAAV.MRTF-shRNA. **(f)** Rentrop-Werte zeigten eine erhöhte Kollateralisierung nach rAAV.Tβ4-Transduktion, außer im Fall von Mitverabreichung von MRTF-A shRNA. **(g)** Regionaler myokardialer Blutfluss bei Flussreserve (Vorhofstimulation 130/min) verbesserte sich in rAAV.Tβ4-behandelten Tieren, aber nicht in rAAV.Tβ4+MRTF-shRNA-Herzen. **(h)** Analyse der Auswurffraktion zeigte eine verbesserte systolische Myokardfunktion in rAAV.Tβ4-transduzierten Tieren (Tag 56), verglichen mit Tag 28 (Tag der Transduktion). Keine Verbesserung der Auswurffraktion wurde in rAAV.Tβ4+MRTF-shRNA-behandelten Herzen beobachtet. **(i)** MRT-Bilder von rAAV.Tβ4-transduzierten Herzen ohne (links) oder mit (rechts) rAAV.MRTF-shRNA-Mitverabreichung. **(j)** Regionale Myokardfunktion, gemessen durch subendokardiale Segmentverkürzung in Ruhe und bei Vorhofstimulation (130 und 150 bpm) zeigt eine erhöhte funktionale Reserve nach rAAV.Tβ4-, aber nicht rAAV.Tβ4+MRTF-shRNA-Transduktion (Durchschnitt ± Stabw., n=5, * p<0,05, ** p<0,001).

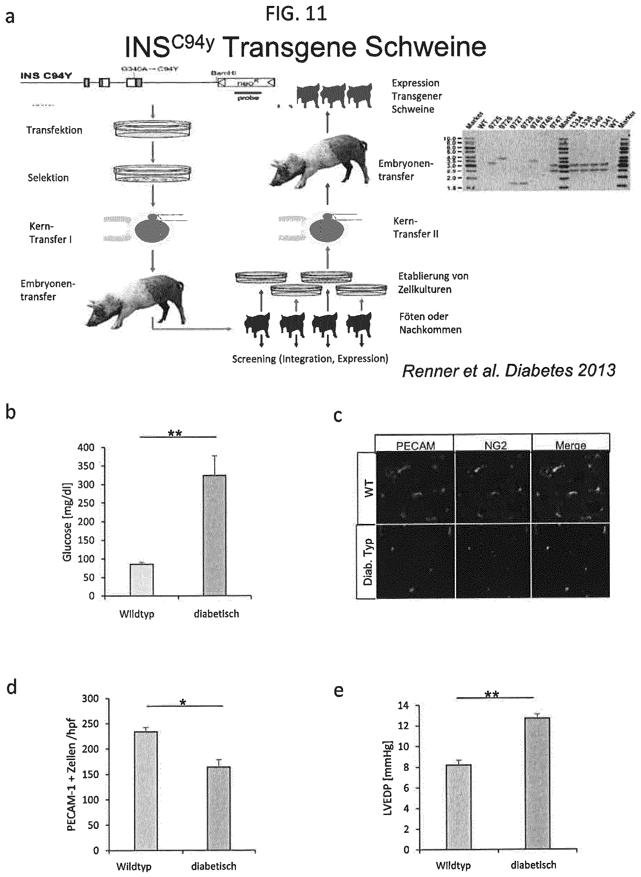

FIG. 11: Herstellung und kardiale Phänotypisierung von INS^{C94Y}-transgenen Schweinen (Diabetes mellitus Typ I)
   **(a)** Ablauf der Herstellung der INS^{C94Y}-transgenen Schweine. **(b)** Blutglucosespiegel von Wildtyp- und diabetischen Schweinen. **(c)** Fluoreszenzfärbung von Endothelzellen (PECAM-1 positive Zellen, rot) und Perizyten (NG-2 positive Zellen, grün). **(d)** Anzahl der Endothelzellen im Myokard von Wildtyp- und diabetischen Schweinen. **(e)** Linksventrikulärer enddiastolischer Druck in Tieren mit Diabetes mellitus Typ I und Wildtyp-Tieren.

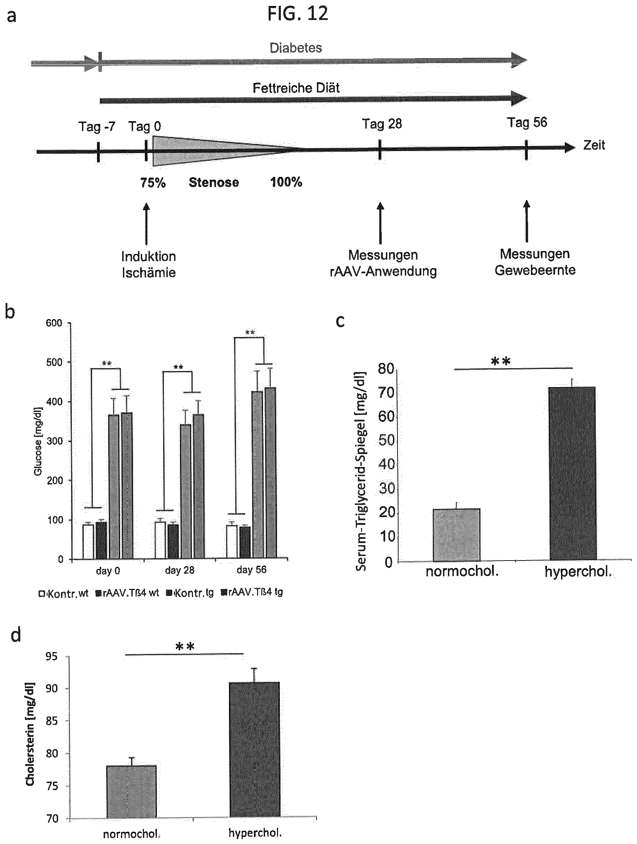

FIG. 12: Charakterisierung des chronisch-ischämischen Myokardmodells mit kardiovaskulären Risikofaktoren
   **(a)** Protokoll des Schweinemodells für hibernierendes Myokard mit Diabetes mellitus Typ I oder Hypercholesterinämie. **(b)** Blutglucosekonzentration der einzelnen Gruppen von Tieren über die Versuchsdauer: Kontrolle Wildtyp; Wildtyp mit rAAV.Tβ4 behandelt; Kontrolle mit Diabetes; Diabetes mit rAAV.Tβ4 behandelt. **(c,d)** Serum-Triglycerid- und Cholesterinspiegel in Tieren mit Hypercholersterinämie (fettreiche Ernährung) und normaler Ernährung.

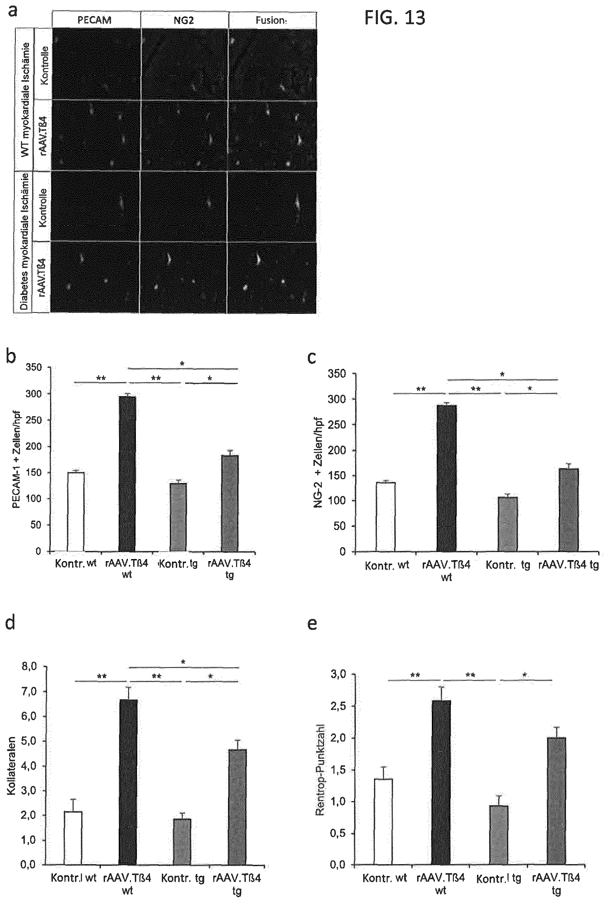

FIG. 13: Einfluss der rAAV.Tβ4-Applikation in Tieren mit Diabetes mellitus Typ I auf Angio- und Arteriogenese
   **(a)** Fluoreszenzfärbung von Endothelzellen (PECAM-1 positive Zellen, rot) und Perizyten (NG-2 positive Zellen, grün) im hibernierenden Schweinemyokard von diabetischen und Wildtyp-Tieren. **(b,c)** Anzahl von Endothelzellen und Perizyten. **(d)** Anzahl der gebildeten Kollateralen. **(e)** Rentrop-Punktzahl.
FIG. 14: Funktionelle Effizienz einer rAAV.Tβ4-Applikation in Tieren mit Diabetes mellitus Typ I
   **(a,b)** Linksventrikulärer end-diastolischer Druck an Tagen 28 und 56 sowie seine Änderung zwischen diesen Zeitpunkten. **(c,d)** Auswurffraktion an Tagen 28 und 56 sowie seine Änderung zwischen diesen Zeitpunkten.
FIG. 15: Einfluss von erhöhten Cholesterinspiegeln auf die Tβ4-vermittelte Angio- und Arteriogenese
   Anzahl von **(a)** Endothelzellen, **(b)** Kollateralen und **(c)** Rentrop-Punktzahl im ischämischen Areal von hypercholesterinämischen Kontroll- und rAAV-Tβ4-behandelten Tieren.
FIG. 16: Funktionelle Effizienz einer rAAV.Tβ4-Applikation in Tieren mit Hypercholesterinämie
   **(a,b)** Linksventrikulärer end-diastolischer Druck an Tagen 28 und 56 sowie seine Änderung zwischen diesen Zeitpunkten in hypercholesterinämischen Kontroll- und rAAV-Tβ4-behandelten Tieren. **(c,d)** Auswurffraktion an Tagen 28 und 56 sowie seine Änderung zwischen diesen Zeitpunkten in hypercholesterinämischen Kontroll- und rAAV-Tβ4-behandelten Tieren. **(e)** Die regionale Myokardfunktion, gemessen als subendokardiale Segmentverkürzung in Ruhe und unter gesteigerter Herzfrequenz (130 und 150 Schläge pro Minute).

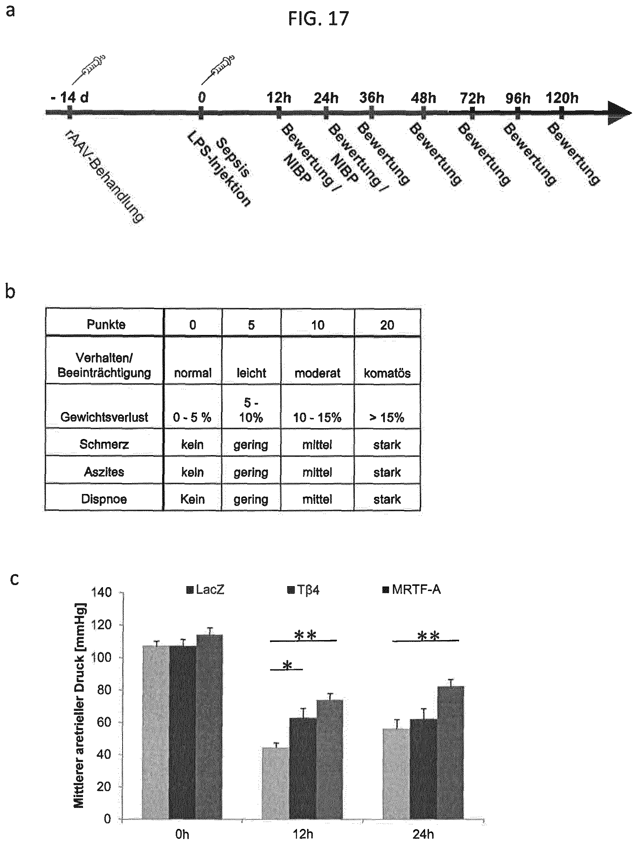

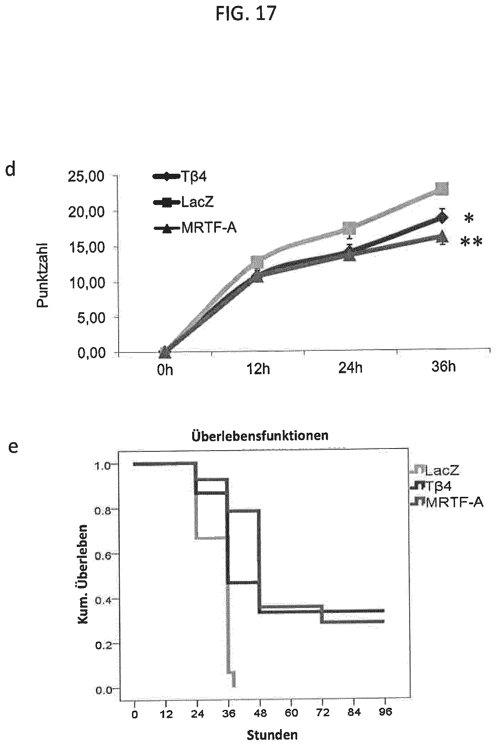

FIG. 17: rAAV.Tβ4- und rAAV.MRTF-A-Vorbehandlung in einem Mausmodell der Sepsis
   **(a)** Protokoll der Sepsis Versuche in Mäusen. **(b)** Punkteschema der Bewertung von Sepsissymptomen in Mäusen und Festlegung der Abbruchkriterien. **(c)** Periphere arterielle Blutdruckwerte nach 12 und 24 Stunden in mit verschiedenen rAAV behandelten Tieren mit Sepsis. **(d)** Punktzahlen der Symptome der Tiere mit Sepsis in den Versuchsgruppen. **(e)** Kumulatives Überleben nach LPS-induzierter Sepsis.

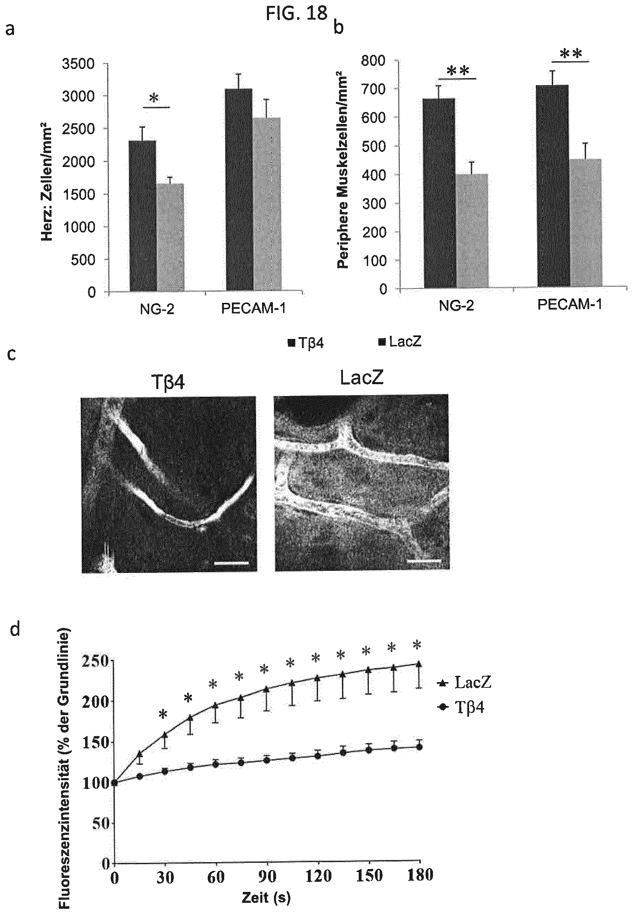

FIG 18: Rolle von MRTF-A und Tβ4 für die vaskuläre Integrität in der Sepsis
   **(a,b)** Histologische Analysen der Endothelzellen (PECAM-1 positive Zellen) und der Perizyten (NG-2 positive Zellen) im Herzen und in der peripheren Muskulatur von Mäusen mit Sepsis. **(c,d)** Beispielbilder und quantitative Analyse für eine Permeabilitätsmessung mittels fluoreszenzmarkiertem hochmolekularem Dextran 6 Stunden nach Sepsisinduktion.

### AUSFÜHRLICHE BESCHREIBUNG DER ERFINDUNG

In unseren Experimenten (siehe Beispiele) haben wir gefunden, dass die Kombination aus einem langwirksamen Vektor und der Überexpression eines wirksamen vasoaktiven Wachstumsfaktors eine Therapieoption für Patienten mit chronisch ischämischen Erkrankungen in Skelett- oder Herzmuskelgewebe darstellt. Die Kombination aus einem Adeno-assoziierten viralen Vektor und Thymosin β4 (Tβ4) bzw. MRTF-A-Transgen führt zu robuster therapeutischer Gefäßneubildung in drei Spezies (Maus, Kaninchen und Schwein). Diese therapeutische Neovaskularisierung wiederum führt in den Modellen der periphären arteriellen Verschlusskrankheit sowie der chronischen myokardialen Ischämie zu einer deutlich verbesserten Perfusion. In dem Modell der chronischen ischämischen Kardiomyopathie am Schwein führt es zudem zu einer gesteigerten Herzfunktion. Dieser spezifische Effekt kann auch noch in Großtieren mit zusätzlichen kardiovaskulären Risikofaktoren (erhöhte Zucker- oder Lipidspiegel) erreicht werden.

Ein Schlüsselmerkmal von MRTF-A-Aktivierung ist Translokation in den Zellkern nach Verringerung von G-Actin-Spiegeln und Export aus dem Kern, wenn die Menge an G-Actin steigt (Miralles et al., Cell 2003, 113:329-42*;* Vartiainen et al., Science 2007, 316:1749-52). Erzwungene Expression von MRTF-A oder Tβ4, einem Peptid, das MRTF-A durch G-Actin-Bindung aktiviert (Fig. 1), leitet eine orchestrierte mikro- und makrovaskuläre Wachstumsantwort im Fall chronischer Ischämie von peripheren (Fig. 3,5) und Herzmuskelzellen (Fig. 7) ein. Übereinstimmend damit wurde chronische Dysfunktion von hibernierendem Schweinemyokard sowohl durch direkte MRTF-A-Überexpression als auch MRTF-A-Aktivierung mittels Tβ4 aufgelöst (Fig. 7). Die Vorstellung, dass MRTF-A-SRF-Signalgebung Myofilamente bereitstellt ist von besonderem Interesse, da ein Verlust des Actin-Zytoskeletts ein Kennzeichen hibernierenden Myokards ist, das durch chronische koronare Hypoperfusion verursacht wird (Bito et al., Circ Res 2007, 100:229-37). Daher befindet sich MRTF-A an der Schnittstelle von Myozyten- und Gefäßregeneration in hibernierendem Myokard. Tβ4, das häufigste G-Actin-bindende Peptid des Zytosols, kann Gefäßwachstum durch endotheliale Migration und Sprossung beeinflussen (Grant et al., J Cell Sci 1995, 108:3685-94*;* Smart et al., Nature 2007, 445:177-82). Eine wesentliche Rolle von MRTF-A in der Tβ4-Signalgebung wurde in vitro und in vivo nachgewiesen, da MRTF-A-shRNA endotheliale Migration und Sprossung (Fig. lb,d) sowie Mikro- und Makrogefäßwachstum (Fig. 3d,f) und funktionale Verbesserung des Herzens (Fig. 10) unterdrücken konnte. Entsprechend verursachte endothelspezifische Defizienz an MRTFs unvollständige Ausbildung des primären vaskulären Plexus in der sich entwickelnden Netzhaut (Weinl et al., J Clin Invest 2013, 123:2193-206). Des Weiteren wurde SRF, das Hauptziel von MRTF-A, kürzlich als wesentlich für das Verhalten von Spitzenzellen in sprossender Angiogenese nach VEGF-A-Stimulation identifiziert (Franco et al., Development 2013, 2321-33*;* Andoh et al., J Biochem 2006, 140:483-9). Nichtsdestotrotz führt VEGF-A zum Wachstum von unreifen und instabilen Kapillaren (Dor et al., EMBO J. 2002, 21:1939-47), im Gegensatz zu Tβ4-MRTF-A, was auf einen Unterschied in der Signalgebung der beiden Gefäßwachstumsfaktoren hinweist.

Zusammengenommen zeigen unsere Daten, dass Aktivierung von Tβ4/MRTF kollateralen Blutfluss im ischämischen Herzen und Hinterbein durch Induktion von CCN1/CCN2 verbessert. Auf der zellulären Ebene geht diese Antwort mit endothelialer Sprossung durch CCN1 (CYR61) und Reifung, d.h. Perizyten-Auskleidung, durch CCN2 (CTGF) einher, was zu einem stabilen und funktionalen Gefäßnetz führt, das kollateralen Blutfluss tragen und das Leitvermögen verbessern kann. Perizyten-Auskleidung ist hierbei wesentlich, da Ang-2 durch Aufheben der Perizyten-Auskleidung (Ziegler et al., J Clin Invest 2013, 123:3436-45) die durch Tβ4-MRTF-Signalgebung ausgeübten positiven Effekte beseitigte (Fig. 5). Dieser Befund zeigt die zentrale Rolle von Gefäßreifung und ausgeglichenem Gefäßwachstum an und bereitet neue therapeutische Wege zu funktionaler Neovaskularisation.

Die Erfindung umfasst daher in einer ersten Ausführungsform einen Adeno-assoziierten viralen Vektor (AAV-Vektor) umfassend ein erstes Gen, das für einen Myocardin-verwandten Transkriptionsfaktor A (MRTF-A) kodiert. AAV-Vektoren sind hierbei Partikel, die die Hülle eines Adeno-assoziierten Virus aufweisen, während sie in ihrem Inneren eine einzelsträngige DNS umfassen, die ein Gen von Interesse kodiert. Das Gen von Interesse kann durch die Infektion einer Zielzelle mit dem AAV-Vektor in die Zielzelle eingeschleust werden.

Das MRTF-A kann aus einem Menschen, einer Maus, einem Kaninchen, einem Schwein, oder jedem anderen Säugetier stammen.

Besonders bevorzugt ist die Verwendung eines AAV-Vektors, der Hüllproteine, insbesondere das cap-Protein, aus AAV9 aufweist. AAV9 weist einen Herzmuskeltropismus auf und bietet daher eine homogene und stabile Expression im Herzmuskel einer Vielzahl von Spezies. Es kann jedoch auch ein mit AAV9 pseudotypisierter AAV-Vektor verwendet werden. Hierunter versteht man einen Vektor, der Hüllproteine von AAV9 aufweist, ansonsten jedoch Proteine aus einem anderen Stamm exprimiert und auch genomische Elemente, zum Beispiel interne terminale Wiederholungen (ITRs) aus diesem anderen Stamm enthält. Z.B. ist AAV2.9 ein mit Hüllproteinen von AAV9 pseudotypisierter AAV2-Vektor. Für die vorliegende Erfindung bieten sich AAV2.9, AAV1.9 und AAV6.9 als pseudotypisierte Vektoren an. Durch Verwendung eines Herzmuskel-tropischen Vektors wird sichergestellt, dass die Expression von MRTF-A im Herzmuskel erfolgt, wo sie die therapeutische Neovaskularisierung einleiten kann.

Alternativ kann, insbesondere zur Behandlung von peripherer Ischämie, auch ein AAV-Vektor mit Skelettmuskeltropismus verwendet werden. Beispiele hierfür sind AAV6, AAV1, AAV9, oder mit diesen Stämmen pseudotypisierte Vektoren.

Das MRTF-A-Gen im erfindungsgemäßen Vektor steht vorzugsweise unter Kontrolle eines kardiospezifischen Promotors, d.h. eines Promotors, der Expression hauptsächlich im Herzmuskel ermöglicht. Beispielhafte kardiospezifische Promotoren sind der MLC2-Promotor, der α-Myosin-schwere Kette-Promotor (α-MHC-Promotor) und der Troponin-I-Promotor (TnI-Promotor). Es können jedoch auch andere, konstitutive oder induzierbare, Promotoren verwendet werden, z.B. ein CMV-Promotor oder ein MyoD-Promotor. Das MRTF-A-Gen kann auch unter der Kontrolle von mehreren Promotoren stehen.

Verfahren zur Herstellung von AAV-Vektoren zum Transfer von spezifischen Genen von Interesse sind im Stand der Technik bekannt (s. z.B. Bell et al., J Clin Invest 2011, 121:2427-35). Ein Verfahren besteht in der Tripel-Transfektion einer geeigneten Produzenten-Zelllinie, z.B. U293, und anschließender Aufreinigung über Cäsiumchlorid-Gradienten, wie im Abschnitt "Material und Methoden" nachfolgend beschrieben. Hierbei werden die Produzenten-Zellen mit drei Vektoren transfiziert: Auf einem ersten Vektor ist das Gen von Interesse kodiert, flankiert von entsprechenden Verpackungssignalen; auf einem zweiten Vektor sind die benötigten AAV-Proteine, insbesondere rap und cap, kodiert; und ein dritter Vektor stellt adenovirale Helferfunktionen bereit, ohne die eine AAV-Partikelproduktion nicht möglich ist.

In einer weiteren Ausführungsform betrifft die Erfindung auch eine pharmazeutische Zusammensetzung umfassend den erfindungsgemäßen Vektor und einen pharmazeutisch akzeptablen Träger. Die pharmazeutische Zusammensetzung kann für jede Art der aus dem Stand der Technik bekannten Verabreichung bestimmt sein. Bevorzugt sind Zusammensetzungen zur intravenösen oder intramuskulären Injektion. Die pharmazeutische Zusammensetzung kann zusätzlich Salze, Puffer, Stabilisatoren, Farbstoffe, Bindemittel, Geschmacksstoffe, usw. umfassen.

Die Erfindung richtet sich auch auf den erfindungsgemäßen AAV-Vektor oder die erfindungsgemäße pharmazeutische Zusammensetzung zur Verwendung als Medikament. Insbesondere kann die Verwendung in einem Säugetier zur Behandlung koronarer Herzerkrankungen oder peripherer Ischämie erfolgen. Bevorzugte Säugetiere sind Mensch, Schwein, Kaninchen und Maus.

Hierbei wird unter einer "koronaren Herzerkrankung" wird eine Erkrankung der Herzkranzgefäße verstanden. Die koronare Herzerkrankung kann myokardiale Ischämie, akuter Herzinfarkt (Myokardinfarkt), stabile Angina pectoris und/oder hibernierendes Myokard sein, aber auch Herzrhythmusstörungen und/oder Herzinsuffizienz. Bei einer "peripheren Ischämie" handelt es sich um eine Minderdurchblutung oder ein vollständiger Durchblutungsausfall eines Gewebes oder Organs außerhalb des Herzens, während eine "myokardiale Ischämie" den Herzmuskel selbst betrifft.

Besonders geeignet sind die erfindungsgemäßen Vektoren zur Behandlung von sogenannten "No-Option"-Patienten. In solchen Patienten sind alle interventionellen und chirurgischen Möglichkeiten einer Therapie ausgeschöpft. Im Allgemeinen wird versucht, die Progression der Erkrankung mittels medikamentöser Therapie zu verlangsamen. Diese zielt jedoch auf eine Lipidsenkung und Plättchenhemmung, nicht jedoch auf eine Gefäßneubildung. Therapeutische Neovaskularisation kann diese Hürde überwinden, sofern molekulare Signalwege verwendet werden, die zu einer balancierten Gefäßneubildung führen. MRTF-A und auch Tβ4 sind zwei Moleküle, die diese Art der balancierten Gefäßneubildung (Kapillaren, Mikrogefäßreifung und Kollateralenbildung) im ischämischen Gewebe induzieren, und dies bei gleichzeitigem Fehlen von ungewollten Nebenwirkungen.

Ferner sind erfindungsgemäße Vektoren besonders geeignet zur Behandlung von Subjekten, die mit zusätzlichen kardiovaskulären Risikofaktoren belastet sind. Hierbei kann es sich um Diabetes mellitus handeln, insbesondere Diabetes mellitus Typ I oder Typ II. Der Risikofaktor kann auch eine erhöhte Konzentration von Cholesterin im Blut (Hypercholesterinämie), wie sie zum Beispiel durch zu fettreiche Ernährung verursacht werden kann, sein. Die erhöhte Cholesterinkonzentration kann eine erhöhte LDL-Cholesterinkonzentration oder eine erhöhte HDL-Cholesterinkonzentration sein.

### BEISPIELE

### Beispiel 1: Induktion von Kennzeichen von Angiogenese durch MRTF-A in vitro

Wir fanden (Fig. 1a-d), dass MRTF-A Kennzeichen von Angiogenese induzierte, d.h. Migration und Tubusbildung von kultivierten menschlichen mikrovaskulären Endothelzellen, in einem ähnlichen Ausmaß wie Tβ4. Der proangiogene Effekt von MRTF-A war abhängig von dem G-Actin-Bindemotiv von Tβ4, da Mutation dieser Domäne und Aufhebung der G-Actin-Bindung den Effekt von Tβ4 auf das Gefäßwachstum beseitigte, ebenso wie eine shRNA, die zugleich die Transkription von MRTF-A und -B störte (MRTF-shRNA; Leitner et al., J Cell Sci 2011, 124:4318-31). Übereinstimmend dazu erhöhte Tβ4 die MRTF-A-Translokation in den Zellkern (Fig. le, Fig. 2a-b), ebenso wie die Transkription eines MRTF/SRF-abhängigen Reportergens, das drei SRF-Bindungsstellen des c-fos-Promotors enthielt (p3DA.Luc, Fig. 1f; Geneste et al., J Cell Biol 2002, 157:831-8). Sowohl MRTF-A als auch Tβ4 induzierten die Expression von Genen, die in Mikrogefäßwachstum involviert sind, insbesondere CCN1, das Angiogenese vermittelt (Hanna et al., J. Biol. Chem. 2009, 284:23125-36), und CCN2, das für die Attraktion von 10T1/2-perizytenartigen-Zellen relevant ist (Fig. 2c-g; Hall-Glenn et al., PLoS ONE 2012, 7:e30562). Wir bemerken, dass Tβ4-Transfektion den MRTF-A gehalt nicht beeinflusste (Fig. 2h), im Gegensatz zu MRTF-A-Transfektion. Übereinstimmend damit, dass CCN1/2 MRTFs nachgeschaltet und für Gefäßbildung relevant sind, verhinderte Störung mit CCN1-shRNA Tβ4-induzierte Tubusbildung (Fig. 1g), wohingegen CCN2-shRNA die Anheftung einer murinen perizytenartigen Zelllinie (C3H/10T1/2) an endotheliale Tubi in vitro unterbrach (Fig. 1i-j).

### Beispiel 2: Behandlung von Hinterbeinischämie in der Maus mit AAV-basierter MRTF-A-Gentherapie

Um die Relevanz von MRTF-A-Signalgebung in vivo weiter zu zeigen, verwendeten wir ein Mausmodell mit Hinterbeinischämie. Intramuskuläre Injektion von rekombinanten AAV-Vektoren (rAAV, Fig. 4a-c) erhöhte die Gewebekonzentration von Zielproteinen in dem behandelten Bein (Fig. 3a) und Transkriptspiegel der nachgeschalteten Mediatoren CCN1 und CCN2 (Fig. 3b; Fig. 4d-f). In Übereinstimmung dazu induzierte rAAV.MRTF-A Kapillarwachstum (Fig. 3c,d) und erhöhte die Perfusion an Tag 7 (Fig. 3e-f). Als ein vorgeschalteter Aktivator hatte Tβ4 einen ähnlichen Effekt auf Gefäßwachstum und -funktion (Fig. 2c-f), sofern das G-Action-Bindemotiv nicht fehlte (Tβ4m) oder eine rAAV.MRTF-shRNA mitverabreicht wurde. Dieser Vektor kodiert eine shRNA, die sowohl gegen MRTF-A als auch MRTF-B gerichtet ist und die Sequenz 5'-GAUCCCCGCAUGGAGCU GGUGGAGAAGAAUUCAAGAGAUUCUUCUCCACCAGCUCCA UGUUUUUGGAAA-3' (SEQ ID NO:1) hat. Um die Relevanz von MRTFs in Tβ4-induziertem Gefäßwachstum weiter zu untersuchen, wurde rAAV.Cre an *Mrifa*^{-/-}*-Mrtfb*^{*flox*/*flox*}-Hinterbeinen verabreicht, um MRTF-A/B-Doppeldefizienz zu erzeugen. In Cre-induzierten MRTF-A/B-Knockouts war Tβ4 nicht in der Lage, an Tag 7 nach Ischämieinduktion Kapillarwachstum (Fig. 3g) und Perizytenrekrutierung zu stimulieren (Fig. 4g-h) und die Perfusion zu verbessern (Fig. 3h, Fig. 4i). Ähnlich zeigten Hinterbeine, wenn rAAV.Cre an CCN1^{flox/flox}-Mäuse verabreicht wurde, keine Tβ4-vermittelten Steigerungen von Kapillarien (Fig. 3i,j) oder Perfusion an Tag 7 (Fig. 3k,l). Daher stimuliert MRTF-A-Transduktion oder MRTF-Aktivierung über Tβ4-vermittelte G-Actin-Sequestrierung Transkription von CCN1, um funktionale vaskuläre Regenration zu vermitteln.

### Beispiel 3: Behandlung von Hinterbeinischämie im Kaninchen mit AAV-basierter MRTF-A-Gentherapie

Die wechselseitige Abhängigkeit von Mikrogefäßwachstum und Arteriogenese für die Vermittlung von für die Wiederherstellung des Durchflusses wurde in einem Kaninchenmodell mit ischämischen Hinterbeinen untersucht (Fig. 6a), welches mit topischer Trennung des Mikrogefäßwachstum-Bereichs (Unterschenkel) und des Kollateralisierungs-Bereichs (Oberschenkel) vereinbar ist. Regionale Transduktion ischämischer Wadenmuskel mit MRTF-A oder Tβ4 (Fig. 5a, Fig. 6b-d) führte zu funktionaler Neovaskularisierung einschließlich CD31⁺-Kapillarsprossung (Fig. 5b,c), NG2⁺-Perizyten-Auskleidung (Fig. 5b,d) und kollateralem Wachstum (Fig. 5e,f). Insbesondere erhöhte MRTF-Aktivierung über Tβ4-Transduktion der Hüftregion, obwohl sie in der Lage war, mäßiges kollaterales Wachstum zu induzieren, nicht die Perfusion, wohingegen Beschränkung der MRTF-Aktivierung über Tβ4 auf die Wadenregion genügte, um mikro- und makrovaskuläres Wachstum sowie Perfusion signifikant zu stimulieren (Fig. 6e-i). Ablösung von mikrovaskulären Perizyten durch erzwungene Angiopoietin-2-Expression (Fig. 5b-d) hob Tβ4-vermittelte Kollateralisierung und Durchflussverbesserung auf (Fig. 5e-g). Außerdem beeinflusste Blockierung von Flussinduzierter Vasodilatation durch orale Verabreichung von L-NAME, einem nichtselektiven Stickoxidsynthase-Hemmer, Kapillarwachstum und -reifung nicht (Fig. 6j), aber verhinderte die Bildung von Kollateralen und erhöhte Perfusion (Fig. 6k,l). Daher scheint Stickoxid, auf Mikrogefäßwachstum und -reifung folgend, Kollateralenwachstum zu vermitteln. Diese Beobachtung wird ergänzt durch den Befund, dass direkte Tβ4-Injektion in das Gebiet des Kollateralenwachstums (Oberschenkel) Perfusion nicht in dem Ausmaß verbessert wie entfernte Injektion von rAAV.Tβ4 in den Unterschenkel, der Stelle des Mikrogefäßwachstums (Fig. 6e-i). Diese Befunde deuten an, dass Mikrogefäßreifung und Stickoxid-Signalgebung Prozesse sind, die in der Abfolge von MRTF-A-vermitteltem Gefäßwachstum stattfinden müssen, um funktionale Neovaskularisierung zu erreichen.

### Beispiel 4: Behandlung von hibernierendem Myokard im Schwein mit AAV-basierter MRTF-A-Gentherapie

Obwohl periphere und koronare Arterien beide Muskelgewebe perfundieren, ist seine permanente Kontraktionsaktivität ein einzigartiges Merkmal des Herzmuskels, welche eine fortwährende Sauerstoffzufuhr benötigt. Ein chronisches Abfallen der Sauerstoffzufuhr verändert die zelluläre Zusammensetzung von lebenden Kardiomyozyten in dem ischämischen Gebiet, was zu einem regionalen Verlust von Kontraktionskraft führt, die hibernierendes Myokard genannt wird (Heusch und Schulz, J Mol Cell Cardiol 1996, 28:2359-72*;* Nagueh et al., Circulation 1999, 100:490-6). Innerhalb von Kardiomyozyten sind Kennzeichen von hibernierendem Myokard ein reduzierter Gehalt an Myofilamenten *(*Bito et al., Circ Res 2007, 100:229-37) und Mitochondrien sowie ein erhöhter Glykogengehalt (St. Louis at al., Ann Thoracic Surg 2000, 69:1351-7). Hier untersuchten wir das Potenzial von MRTF-A, Dysfunktion in hibernierendem Myokard aufzulösen, die durch perkutane Implantation eines Reduktionsstents in Schweineherzen erzeugt wurde *(*Kupatt et al., J Am Coll Cardiol 2007, 49:1575-84), was zu einem allmählichen Verschluss des Ramus Circumflexus führt (RCx, Fig. 8a). An Tag 28 nach rAAV.MRTF-A-Verabreichung in das ischämische Gebiet, was den MRTF-A-Gehalt im Gewebe signifikant erhöhte (Fig. 8b), fanden wir einen signifikant höheren Grad an Kapillardichte und Perizytenbedeckung (Fig. 7a-c). Kollateralenwachstum und Perfusion unter schneller Herzfrequenz (130/min) waren an Tag 28 immer noch beeinträchtigt, d.h. vor LacZ- und MRTF-A-Transduktion (Fig. 8c-f), aber verbesserten sich an Tag 56, d.h. 4 Wochen nach MRTF-A-Transuktion, aber nicht nach LacZ-Transduktion (Fig. 7d-f).

Erhöhte kollaterale Perfusion (Fig. 8g) erzeugte eine verbesserte funktionelle Reserve des ischämischen Gebiets bei schneller Herzfrequenz (130 und 150 Schläge pro Minute, Fig. 7g). Zugleich fanden wir eine verbesserte Auswurffraktion als Marker globaler systolischer Funktion (Fig. 7h) und einen Abfall des enddiastolischen Drucks in der linken Herzkammer (Fig. 8i), einem prognostischen Marker beginnenden Herzversagens.

Transgene Schweine, die Tβ4 ubiquitär und konstitutiv exprimieren (Fig. 9), zeigten ähnliches Kapillarwachstum und -reifung (Fig. 7a-c). An Tag 56 war die Blutflussreserve im ischämischen Gebiet erhöht (Fig. 7f) und die funktionelle Reserve der ischämischen Region (Fig. 7g) oder des ganzen Herzens (Fig. 7h) waren erhöht wie in rAAV.MRTF-A-behandelten Herzen. Insbesondere erfuhren auf Grund der konstitutiven Tβ4-Überexpression von Tag 0 bis Tag 28 Tβ4tg-Tiere keinen signifikanten Verlust von Perfusion oder myokardialer Funktion in Ruhe oder bei schneller Herzfrequenz (Fig. 7g, Fig. 8d-g,i).

Des Weiteren wurden rAAV.Tβ4-induziertes Mikro- und Makrogefäßwachstum und darauffolgende Erhöhungen der Perfusionsreserve unterdrückt, wenn eine inhibitorische MRTF-A-shRNA mitverabreicht wurde (Fig. 10a-f). Der Gewinn an globaler (Fig. 10h, Beispiele in Fig. 10i) und regionaler Myokardfunktion (Fig. 10j) wurde beseitigt, wenn Tβ4-Transduktion mit MRTF-A-Inhibierung durch eine geeignete shRNA kombiniert wurde.

Hier zeigen wir unter Verwendung eines kombinierten genetischen und physiologischen Ansatzes in Maus-, Kaninchen- und Schweinemodellen, dass MRTFs Wachstum und Reifung von Mikrogefäßen sowie erhöhten kollateralen Blutfluss nach Arterienverschluss in Hinterbein- und koronaren Netzen stimulieren. Mechanistisch zeigen wir, dass Thymosin β4 nachgelagerte MRTF-Translokation SRF koaktiviert und CCN1/CCN2 induziert, was zu erhöhter Angiogenese und Rekrutierung von vaskulären glatten Muskelzellen und Bildung von funktionalen Gefäßen führt, die kollateralen Fluss tragen können (Fig. 7i).

### Beispiel 5: Behandlung von hibernierendem Myokard in diabetischen Schweinen mit AAV-basierter Tβ4-Gentherapie

### Herstellung und kardiale Phänotypisierung von INS^{C94Y} transgenen Schweinen (Diabetes mellitus Typ I)

Die Herstellung transgener Schweine mit der Mutation C94Y im Insulin-Gen (INS^{C94Y}) ist in Figur 11 dargestellt. Diese Mutation ist auch in Renner et al., Diabetes 2013, 62:1505-1511 beschrieben. Die C94Y-Mutation führt zu einer Missfaltung des Insulinproteins in den β-Zellen des Pankreas und einer Ansammlung des missgefalteten Insulins im endoplasmatischen Reticulum (ER). ER-Stress führt zu β-Zell-Apoptose und dadurch schließlich zu Diabetes mellitus Typ I.

Zunächst wurde in Schweine-Fibroblasten ein INS^{C94Y}-Expressionsvektor mittels Nukleotransfektion eingebracht. Nach Selektion der Fibroblasten wurde eine erste Runde somatischer Kerntransfer in Eizellen durchgeführt. Anschließend wurden die Nachkommen mittels Southern Blot analysiert und die Tiere mit erhöhten Blutglucosespiegeln und verzögertem Wachstum für erneute Klonierung verwendet (siehe auch Renner *et al.* 2013). Diese Tiere wurden dann in einem Alter von 3-4 Monaten für die nachfolgenden Untersuchungen verwendet.

Nach Absetzen der Insulinbehandlung zeigten die Tiere einen deutlich erhöhten Blutglucosespiegel (Fig. 11c & d). Analysen des Herzgewebes für Endothelzellen (PECAM-1 positive Zellen, rot) und für Perizyten (NG-2 positive Zellen, grün) zeigten bereits ohne zusätzlichen Stress eine deutliche Reduktion der Endothelzell- und Perizytenzahl. Die Analyse des links-ventrikulären enddiastolischen Drucks zeigt in Tieren mit Diabetes mellitus Typ I eine signifikante Erhöhung als Ausdruck einer verminderten globalen Herzfunktion bereits in einem frühen Stadium (Fig. 11 e; Durchschnitt ± Stabw.; n=4, * p<0.05, **p<0.001).

Fig. 12 zeigt weitere Auswirkungen von Diabetes mellitus Typ I oder einer fettreichen Diät auf das Myokard von Schweinen. Fig. 12 a veranschaulicht das Testprotokoll des Schweinemodells für hibernierendes Myokard mit Diabetes Mellitus Typ I oder Hypercholesterinämie. Im Vergleich zu den Kontrollgruppen (wt ± rAAV.Tβ4) zeigten die INS^{C94Y}-transgenen Tiere mit Diabetes mellitus Typ I (Kontrolle tg und rAAV.Tβ4 tg) über die gesamte Versuchsdauer erhöhte Blutglucosespiegel (Fig. 12 b). Zwischen der mit rAAV.Tβ4 behandelten Gruppe und der Kontrollgruppe zeigte sich jedoch weder für die Wildtyp-Gruppe noch für die transgenen Tiere ein Unterschied (Fig. 12c, d). Ein Einfluss von Tβ4 oder MRTF-A auf den Blutglucosespiegel ist auch nicht zu erwarten. In den Tieren mit Hypercholesterinämie zeigten sich nach 9 Wochen fettreicher Fütterung deutlich erhöhte Triglycerid- und Cholersterinwerte im Serum (Durchschnitt ± Stabw.; n=4, **p<0.001).

### Wirkung der rAA V. Tβ4-Applikation in Tieren mit Diabetes mellitus Typ I

Im hibernierenden Schweinemyokard induziert eine rAAV.Tβ4-Transduktion eine Kapillarsprossung (PECAM-1-Färbung, rot) und eine Perizytenrekrutierung (NG-2 Färbung, grün) in beiden Gruppen (Wildtyp und Diabetes); Fig. 13a-c. Des Weiteren wurde durch die Überexpression von Tβ4 mittels rAAV ein deutliches Kollateralenwachstum induziert (Fig. 13 d) und es konnte eine deutlich bessere Füllung des distalen Blutgefäßes mittels Rentrop-Punktzahl gemessen werden (Fig. 13 e). Auch hier konnte der Effekt in beiden Gruppen, Wildtyp und Diabetes, gemessen werden (Durchschnitt ± Stabw.; n=4, * p<0.05, **p<0.001).

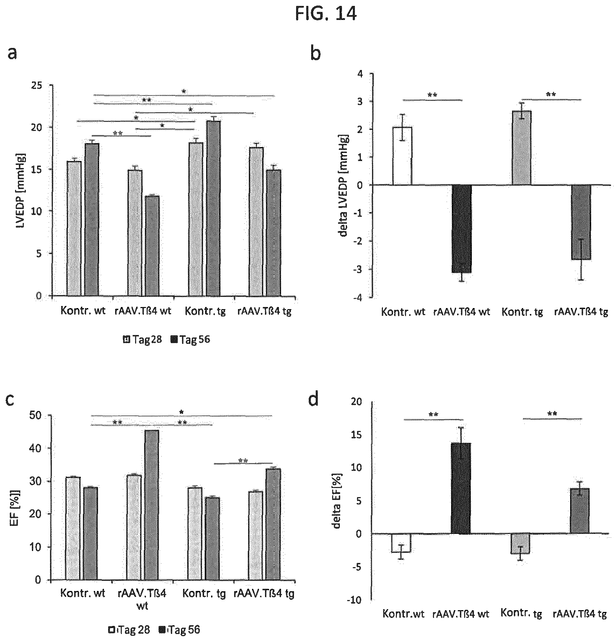

Der linksventrikuläre end-diastolische Druck, ein Parameter für die globale Myokardfunktion, welcher in den Kontrolltieren beider Gruppen von Tag 28 zu Tag 56 weiter anstieg, war in den Tieren mit rAVV.Tβ4-Transduktion deutlich erniedrigt (FIG. 14a, b). Die Auswurffraktion, ein weiterer Parameter für die globale Myokardfunktion, zeigte bei den Kontrolltieren eine weitere Erniedrigung der Werte von Tag 28 zu Tag 56, wohingegen sich der Wert nach Tβ4-Überexpression in beiden Gruppen (Wildtyp und Diabetes) deutlich verbesserte (FIG. 14c, d; Durchschnitt ± Stabw.; n=4, * p<0.05, **p<0.001).

### Beispiel 6: Wirkungen von Tβ4-Gentherapie im Myokard von Schweinen mit Hypercholesterinämie

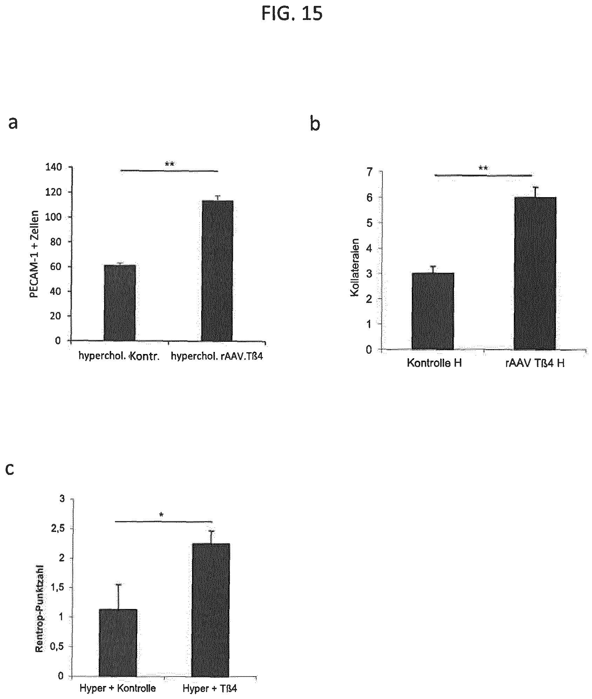

In Kontrolltieren mit 9 Wochen fettreicher Fütterung zeigte sich eine deutliche Reduktion der Kapillaren (PECAM-1 positiven Zellen) im ischämischen Areal (Fig. 15 a). Durch die rAAV.Tβ4-Applikation konnten die Kapillaren (PECAM-1 positiven Zellen) im ischämischen Areal deutlich gesteigert werden. Durch die rAAV.Tβ4-Transduktion konnte auch in Tieren mit erhöhten Cholesterinspiegeln das Kollateralenwachstum gesteigert werden (Fig. 15 b). Dies führte auch zu einer besseren Füllung des distalen Gefäßabschnitts in dem ischämischen Areal, wie in der Rentrop-Punktzahl gezeigt (Fig. 15 c; Durchschnitt ± Stabw.; n=4, * p<0.05, **p<0.001).

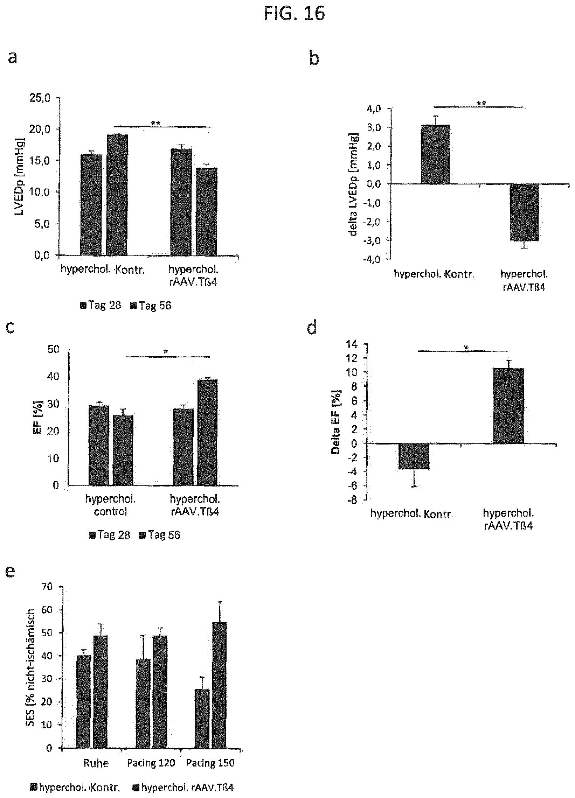

Der linksventrikuläre end-diastolische Druck, ein Parameter für die globale Myokardfunktion, welcher in den Kontrolltieren von Tag 28 zu Tag 56 weiter anstieg, war in den Tieren mit rAVV.Tβ4-Transduktion deutlich erniedrigt (Fig. 16a, b). Die Auswurffraktion, ein weiterer Parameter für die globale Myokardfunktion, zeigte bei den Kontrolltieren einen Abfall der Werte von Tag 28 zu Tag 56, wohingegen sich der Wert nach Tβ4-Überexpression deutlich verbesserte (Fig. 16c, d). Die regionale Myokardfunktion, gemessen als subendokardiale Segmentverkürzung in Ruhe und unter gesteigerter Herzfrequenz (130 und 150 Schläge pro Minute) zeigte eine verbesserte Funktionsreserve bei Tieren mit einer rAAV.Tβ4-Therapie (Fig. 16 e; Durchschnitt ± Stabw.; n=4, * p<0.05, **p<0.001).

### Beispiel 7: Rolle von MRTF-A und Tβ4 für die vaskuläre Integrität in Mäusen mit Sepsis

Fig. 17 a zeigt das Protokoll der Sepsisversuche in Mäusen. Sepsis wurde 14 Tage nach rAAV-Behandlung (rAAV.MRTF-A oder rAAV.Tβ4) durch Injektion von LPS induziert. Zu sieben Zeitpunkten nach der Sepsis-Induktion (12 h, 24 h, 36 h, 48 h, 72 h, 96 h, 120 h) wurde eine Bewertung der Symptome an Hand der in Fig. 17b dargestellten Tabelle vorgenommen. Die Transduktion von MRTF-A oder Tβ4 mittels rAAV vor Induktion der Sepsis führt zu erhöhten peripheren arteriellen Blutdruckwerten nach 12 und 24 Stunden (Fig. 17 c). Im Verlauf bis 36 Stunden nach Sepsis zeigen die mit rAAV.Tβ4 und rAAV.MRTF-A behandelten Tiere im Vergleich zu den mit rAAV.LacZ behandelten Kontrolltieren deutlich niedrigere Symptom-Punktzahlen (Fig. 17 d). Das kumulative Überleben nach LPS-induzierter Sepsis ist durch die Überexpression von Tβ4 und MRTF-A deutlich verbessert (Fig. 17 e; Durchschnitt ± Stabw.; n=7-15, * p<0.05, **p<0.001).

Histologische Analysen der Endothelzellen (PECAM-1 positive Zellen) und der Perizyten (NG-2 positive Zellen) zeigten im Herzen und in der peripheren Muskulatur eine erhöhte Zellzahl (Fig. 18a, b) in mit Tβ4 behandelten Tieren im Vergleich zu Kontrolltieren, die mit rAAV.LacZ transduziert wurden. Beispielbilder und eine quantitative Analyse für eine Permeabilitätsmessung mittels fluoreszenzmarkiertem hochmolekularem Dextran 6 Stunden nach Sepsis Induktion sind in Fig. 18c und d gezeigt. Hierbei zeigte sich ein deutlich verringerter Austritt des Indikators nach Tβ4-Überexpression im Vergleich zu den mit LacZ-transduzierten Kontrolltieren (Durchschnitt ± Stabw.; n=4, * p<0.05, **p<0.001).

### Material und Methoden

Die in den Beispielen beschriebenen Experimente wurden unter Verwendung der nachfolgend beschriebenen Techniken durchgeführt.

### Reagenzien

Alle Zellkulturmedien und Chemikalien wurden von SIGMA (Deisenhofen) erworben, falls nicht anders angegeben. Kontrastmittel Solutrast 370 wurde von Byk Gulden (Konstanz) geliefert.

### Adeno-assoziierte virale Vektoren

Die rekombinanten Vektoren rAAV.MRTF-A, rAAV.Tβ4, r.AAV.Tβ4m, rAAV.LacZ, rAAV.Cre und rAAV.MRTF-shRNA wurden mittels Tripeltransfektion von U293-Zellen hergestellt. Ein Plasmid kodierte für das Transgen unter der Kontrolle eines CMV-Promotors, der von cis-wirkenden internen terminalen Wiederholungen von AAV2 flankiert wurde. Im Fall von rAAV.MRTF-A war dies das Plasmid pAAV-CMV-mMRTF-A (SEQ ID NO:16). Es kann jedoch auch ein Plasmid, das menschliches MRTF-A kodiert, verwendet werden, z.B. pAAV-CMV-hMRTF-A (SEQ ID NO:17). Ein zweites Plasmid stellte AAV2 rep und AAV9 cap in trans bereit (Bish et al., Hum. Gene Ther. 2008, 19:1359-68), während ein drittes Plasmid (delta F6) adenovirale Helferfunktionen ergänzte. Zellen wurden 48 Stunden später geerntet und Vektoren mittels Cäsiumchlorid-Gradienten aufgereinigt wie vorher beschrieben (Lehrke et al., Cell Metab 2005, 1:297-308). Virale Titer wurden mittels Echtzeit-PCR gegen den polyA-Schwanz des bGH des Vektors (Primersequenzen s. Tabelle 1). Trans- und Helferplasmide wurden freundlicherweise von James M. Wilson, University of Pennsylvania, bereitgestellt.

### Zellkultur

SatisFection (TPP AG, Trasadingen, Schweiz) wurde für die Transfektion humaner mikrovaskulärer Endothelzellen (HMECs), muriner Endothelzellen (bEnd.3) und der myozytischen Zelllinie HL-1 gemäß den Anweisungen des Herstellers verwendet. 100 µl Serum- und Antibiotika-freies DMEM-Medium wurden mit 3 µl SatisFection-Transfektionsreagens gemischt.

### In-vitro-Tubusbildung und Kokultivierungs-Experimente

Für Matrigel-Experimente wurden HMECs mit pcDNA, MRTF-A, Tβ4±MRTF-shRNA, Tβ4m (welchem das G-Actin-Bindemotiv KLKKTET fehlte; Bednarek et al., J. Biol. Chem. 2008, 283:1534-44) oder Tβ4±CCN1-shRNA transfiziert. Die Zellen (8000 Zellen pro Loch) wurden auf Matrigel (BD Matrigel™ Basement Membrane Matrix, BD Biosciences, San Jose, USA) in Basalendothel-Wachstumsmedium mit einer Zugabe von 5% fötalem Kälberserum ausgesät und Aufnahmen wurden nach 18 h gemacht. Die Anzahl von Ringen pro Niedrigleistungsfeld wurde quantifiziert.

In Kokultivierungs-Experimenten wurden HL-1-Zellen mit r.AAV.Tβ4±CCN1-shRNA, rAAV.MRTF-shRNA oder rAAV.Tβ4m (1x10⁶ AAV6-Partikel pro Zelle) transduziert. HL-1 und in Matrigel eingebettete HMECs (8.000 pro Loch) wurden durch eine semipermeable Membran physisch getrennt. Nach 18 h wurden die HL-1-Zellen entfernt und Ringbildung pro Niedrigleistungsfeld wurde quantifiziert.

CH3/10T1/2-Perizyten-Zellattraktion zu murinen Endothelzellen (bEnd.3) wurde nach Transfektion des endothelialen Kompartiments mit pcDNA, MRTF-A oder Tβ4±CCN2-shRNA mittels SatisFection (Agilent, Böblingen) getestet. Endothelzellen wurden mit DiD (rot, Vybrant®, Life Technologies) gefärbt und auf Matrigel ausgesät (12.000 Zellen pro Loch). Nach 6 h wurden mit DiO (Vybrant®, Life Technologies) gefärbte Perizyten-artige Zellen (2.000 Zellen pro Loch) hinzugegeben und Migration zu den Tubi hin wurde für 2 h erlaubt. Dann wurden mittels konfokaler Lasermikroskopie (Carl Zeiss, Jena) Kokultur-Aufnahmen gemacht.

### Migrationstest

HMECs wurden wie oben beschrieben mit den angegebenen Transgenen transfiziert. 60.000 Zellen wurden in Löchern mit einem streifenartigen Einsatz (ibidi GmbH, Planegg) bis zur Konfluenz gezogen. Nach 48 h wurden die Zellkerne mit Syto62 gefärbt. Dann wurden die Zellen mit 2% PFA fixiert, permeabilisiert und mit einem anti-MRTF-A-Antikörper (Santa Cruz Biotech, Santa Cruz, USA) und einem sekundären Antikörper (Alexa 488-gekoppelt, Invitrogen, Karlsruhe) inkubiert. Aufnahmen wurden mit konfokaler Lasermikroskopie (Carl Zeiss, Jena) gemacht und die mittlere Fluoreszenzintensität des Gebiets von 100 Zellkernen, identifiziert über Syto62, wurde automatisch mit dem LS5-Bildbrowser ausgewertet.

### HPLC-Analyse

Die Bestimmung von Tβ4 wurde durchgeführt wie vorher beschrieben (Huff et al., Ann. N. Y. Acad. Sci. 2007, 1112:451-7). Dabei wurden Gewebeproben zerstört durch Zugabe von 4 M Perchlorsäure mit 1% Thiodiethanol bis zu einer Endkonzentration von 0,4 M. Mischungen wurden homogenisiert, für 30 min bei 4°C inkubiert und für 10 min bei 20.000 g zentrifugiert. Der Überstand wurde durch reverse-Phase-Chromarographie analysiert. In Kaninchen wurden endogenes und exogenes Tβ4 durch Detektion des kaninchenspezifischen Tβ4-Ala unterschieden.

### Luciferase-Test

Um die MRTF-abhängige Luciferase-Aktivität zu bestimmen, wurden HMECs und HL-1-Zellen mit p3DA.Luc (= ein Konstrukt eines synthetischen Promotors mit drei Kopien der c-fos-SRF-Bindungsstelle und einer Xenopus-Typ-5-Actin-TATA-Box plus einer in pGL3 inserierten Transkriptionsstartstelle; Posern et al., Mol. Biol. Cell 2002, 13;4167-78), einem SRF-Reportergen, und 930 ng an pcDNA, Tβ4 oder Tβ4m transfiziert. Vergleichbare Transfektionseffizienzen wurden durch Kotransfektion von 50 ng ptkRL (Renilla Luciferase-Reporter) sichergestellt. Pellets der Zellen wurden erhalten und lysiert, durch Zentrifugation für 10 min bei 4°C und 13.000 rpm weiter gereinigt und für die Bestimmung der Leuchtkäfer-Luciferase-Aktivität und Renilla-Luciferase-Aktivität verwendet. Das Verhältnis Leuchtkäfer/Renilla-Luciferase-Aktivität wurde berechnet.

### RNA-Modulierung und -Detektion

Echtzeit-PCR (RT-PCR) wurden mit SYBR Green-Farbstoff (iQ SBYR Green Supermix, Bio-Rad, München) durchgeführt und auf einem iQ-Cycler (Bio-Rad, München) gemessen. Die Primer sind in Tabelle 1 aufgeführt. Expressionsniveaus wurden zu GAPDH normalisiert und als Vielfaches der pcDNA-Kontrollsituation dargestellt. Das komparative 2-DDCt-Verfahren wurde durchgeführt wie früher beschrieben (Pfosser et al., Cardiovasc Res 2005, 65:728-36).

### Western-Blot-Analyse von MRTF-A

Für die Analyse von Gesamt-MRTF-A-Protein wurden Zellkultur- und Gewebeproben in 1 ml Lysepuffer enthaltend 20 mM Tris, 1 mM EDTA, 140 mM NaCl, 1% Nonidet P-40 (NP-40), 0,005 mg/ml Leupeptin, 0,01 mg/ml Aprotinin, 1 mM PMSF, pH7,5, homogenisiert. 60 µg Gesamtproteinextrakte wurden durch Polyacrylamid-Gelelektrophorese mit 10 % Natriumdodecylsulfat (SDS-PAGE) aufgetrennt. Nach der Elektrophorese wurden die Proteine auf eine PVDF-Membran (Millipore, Billerica, USA) elektrotransferiert, mit 5% fettfreier Milch in PBS-Puffer mit 0,1% Tween 20 (PBS-T) geblockt und über Nacht bei 4°C mit Primärantikörpern gegen MRTF-A (C-19; Santa Cruz Biotech, Santa Cruz, USA) inkubiert. Nach Waschen der Membran wurde sie mit einem Sekundärantikörper (Esel-anti-Ziege-IgG, HRP-konjugiert; Santa Cruz Biotech, Santa Cruz, USA) inkubiert und mit einem Chemilumineszenz-Reagens (ECL; GE Healthcare, Buckinghamshire, England) entwickelt. Für die Analyse des MRTF-A-Proteingehalts im Zellkern bzw. dem Zytosol wurde eine Trennung mit den Ne-Per®-Reagenzien für zytoplasmatische und Zellkern-Extraktion (Thermo Scientific, Rockford, USA) gemäß den Richtlinien des Herstellers durchgeführt. Dann wurde eine Western-Blot-Analyse wie oben beschrieben durchgeführt. Als Kontrollprotein wurden entweder α-Tubulin (6A204; Santa Cruz Biotech, Santa Cruz, USA) oder, für die Zellkern-Fraktion, Lamin B1 (ZL-5; Santa Cruz Biotech, Santa Cruz, USA) verwendet.

### Tierversuche

Tierpflege und alle experimentellen Verfahren wurden unter strenger Befolgung der deutschen und der NIH-Tierrichtlinien durchgeführt und wurden von der Tierschutzkommission der Regierung von Oberbayern genehmigt (AZ 55.2-1-54-2531-26/09, 130/08, 140/07). Alle Tierversuche wurden im Walter-Brendel-Zentrum für Experimentelle Medizin in München durchgeführt.

### Maus-Hinterbein-Ischämie

Einseitige Hinterbeinischämie des rechten Beins wurde in männlichen C56B16-Mäusen gleichen Alters (Charles River, Sulzfeld) sowie in MRTF-A^{+/-}/B^{flox/flox}-, MRTF-A^{-/-}/B^{flox/flox}-, MRTF-A^{+/-}/B^{-/-}Vi- (= MRTF-A-^{+/-}/B^{flox/flox} + 3x10¹² rAAV.cre), MRTF-A^{-/-}/B^{-/-}Vi- (= MRTF-A-^{-/-}/B^{flox/flox} + 3x10¹² rAAV.cre) (Weinl et al., J. Clin. Invest. 2013, 123:2193-226) und CCN1^{-/-}Vi-Mäusen (= Cyr61^{flox/flox} + 3x10¹² rAAV.Cre; hergestellt im Labor von Ralf Adams am Max-Planck Institut für molekulare Biomedizin in Münster) wie vorher beschrieben durchgeführt (Limbourg et al., Nat. Protocols 2009, 4:1737-48). Vor Induktion der Ischämie (Tag -14) wurden 3x10¹² AAV9-Viruspartikel wie beschrieben (Qin et al., PLoS ONE 2013, 8:e61831) intramuskulär in das rechte Bein verabreicht. An Tag 0 wurde das linke Bein einer Scheinoperation unterzogen, wohingegen im rechten Bein die Femoralarterie ligiert wurde. Die Messungen zur postischämischen Blutflusserholung wurden durch Laser-Doppler-Durchflusszytometrie (Moor Instruments, Devon, England) durchgeführt. Messungen wurden direkt vor und nach der Operation, an Tag 3 und an Tag 7 gemacht. Die Ergebnisse sind als Verhältnis von rechtem zu linkem Bein einschließlich Subtraktion des Hintergrund-Gewebewertes angegeben. RT-PCR- und HPLC-Analyse wurden an Tag 5 nach Ischämieinduktion durchgeführt; Gewebe wurde von behandelten und nicht behandelten Beinen entnommen. Analysen der Kapillardichte und der Gefäßreifung wurden an Tag 7 in allen Gruppen mittels PECAM-1- (sc1506, Santa Cruz Biotech, Santa Cruz, USA) und NG2-Färbung (in MRTF-A^{+/-}/B^{flox/flox}-Mäusen; Chemicon, Nürnberg) in gefrorenen Gewebeproben des M. gastrocnemius und M. adductor durchgeführt.

### Kaninchen-Hinterbein-Ischämie

An Tag 0 wurde in Neuseeland-Kaninchen die komplette Femoralarterie des rechten Beins entfernt (Pfosser et al., Cardiovasc. Res. 2005, 65:728-736) und rAAV-Verabreichung (5x10¹² Viruspartikel) wurde mittels intramuskulärer Injektion in das rechte Hinterbein durchgeführt, wie angegeben. An Tag 7 und 35 wurde Angiographie durchgeführt durch Injektion von Kontrastmittel (Solutrast 370, Byk Gulden, Konstanz) in das ischämische Bein mit einem automatischen Injektor (Harvard Apparatus, Freiburg). Des Weiteren wurden fluoreszierende Mikrokugeln (15 µm, Molecular Probes®, Life Technologies, Carlsbad, USA) für Blutflussmessungen im ischämischen und nicht-ischämischen Gewebe verwendet. Für die Blutflussanalyse wurden Gewebeproben wie früher beschrieben verdaut (Thein et al., Comput. Methods Programs Biomed. 2000, 61:11-21*;* Kupatt et al., J Am Coll Cardiol 2010, 56:414-22). Fluoreszenzanalyse wurde mit einem Tecan Saphire 2-Mikrotiterplatten-Leser bei den Emissionswellenlängen 680 nm, 638 nm, 598 nm, 545 nm, 515 nm, 468 nm und 424 nm durchgeführt, abhängig von dem verwendeten Fluoreszenzfarbstoff. Berechnungen wurden wie vorher beschrieben durchgeführt (Lebherz et al., Endothelium 2003, 10:257-65). Die Analyse der Kapillardichte und der Gefäßreifung wurde mittels PECAM-1- (sc1506, Santa Cruz Biotech, Santa Cruz, USA) und NG2-Färbung (in MRTF-A^{+/-}/B^{flox/flox}-Mäusen; Chemicon, Nürnberg) in gefrorenen Gewebeproben des ischämischen und nicht-ischämischen Beins durchgeführt.

### Chronische myokardiale Ischämie in Schweinen

Schweine wurden wie vorher beschrieben anästhesiert und behandelt (*von* Degenfeld et al., J. Am. Coll. Cardiol. 2003, 42:1120-8). Hierzu wurde ein mit einer PTFE-Membran beschichteter Verengungsstent in den proximalen RCx implantiert, was zu einer 75%igen Reduktion des Blutflusses führte. Korrekte Lokalisierung des Stents und Durchgängigkeit des distalen Gefäßes wurden durch die Injektion von Kontrastmittel sichergestellt. An Tag 28 wurden Basislinien-Messungen für die globale Myokardfunktion (enddiastolischer Druck in der linken Herzkammer = LVEDP, Auswurffraktion = EF) und myokardiale Perfusion (fluoreszierende Mikrokugeln, 15 µm, Molecular Probes®) durchgeführt. Dann wurde selektive druckgeregelte Retroinfusion in die das RCx-perfundierte Myokard ableitende große Herzvene für 5x10¹² Viruspartikel rAAV.MRTF-A und rAAV.Tβ4±rAAV.MRTFA-shRNA durchgeführt. An Tag 56 wurden die Messungen für globale Myokardfunktion und Blutfluss wiederholt und die regionale Myokardfunktion des ischämischen und nicht-ischämischen Gebiets wurde ermittelt (in Ruhe und unter schneller Herzstimulation, 130 und 150 bpm).

Eine post-mortem-Angiographie wurde für die Berechnung des Kollateralenwertes und die Analyse mittels Rentrop-Punktzahl (0 = keine Füllung, 1 = Seitenzweig-Füllung; 2 = teilweise Hauptgefäß-Füllung; 3 = vollständige Hauptgefäß-Füllung) durchgeführt. Gewebe wurde für die Analyse des regionalen myokardialen Blutflusses und Immunhistologie entnommen.

### Globale myokardiale Funktion

An Tag 28 und Tag 56 wurde die globale myokardiale Funktion (LVEDP) durch einen Millar-Druckspitzenkatheter (Sonometrics, Ontario, Kanada) untersucht. Ein Angiogramm der linken Herzkammer für globale myokardiale Funktion wurde an Tag 28 und Tag 56 durchgeführt. Die Auswurffraktion wurde durch Planimetrie der endsystolischen und enddiastolischen Angiogrammbilder erhalten (Image J 1.43u, National Institute of Health, USA).

### Regionale myokardiale Funktion

An Tag 56 nach Induktion von Ischämie wurde eine Sternotomie durchgeführt und Ultraschall-Kristalle wurden subendokardial im nicht-ischämischen Bereich (LAD-Kontrollregion) und im ischämischen Bereich (Cx-perfundierte Region) in standardisierter Weise platziert. Subendokardiale Segmentverkürzung (SES, Sonometrics, Ontario, Kanada) wurde in Ruhe und unter erhöhter Herzfrequenz (funktionale Reserve, Frequenz 130 und 150) untersucht und off-line ECG-abhängig ausgewertet.

### Regionaler myokardialer Blutfluss

Die Analyse des regionalen myokardialen Blutflusses wurde an Tag 28 (vor rAAV-Behandlung) und Tag 56 (28 Tage nach AAV-Behandlung) mittels fluoreszenierender Mikrokugeln (Molecular Probes®) durchgeführt. Die Mikrokugeln (15 µm, 5x10⁶ Partikel pro Injektion) wurden über einen Pigtail-Katheter in die linke Herzkammer injiziert. Blutflussmessungen wurden in Ruhe und bei erhöhter Herzfrequenz (130 bpm) durchgeführt. Der Fluoreszenzgehalt wurde mittels eines Tecan Sapphire 2-Mikrotiterplatten-Lesers analysiert und eine Berechnung des regionalen myokardialen Blutflusses wurde durchgeführt, entweder als ml/g Gewebe absolut oder als Verhältnis zu der nicht-ischämischen Region in Ruhe (Blutfluss % nicht-ischämisch; Kupatt et al., J Am Coll Cardiol 2010, 56:414-22).

### Histologie

Gewebeproben des ischämischen und nicht-ischämischen Gebiets wurden auf Kapillardichte (PECAM-1-positive Zellen, rot) und Perizyten-Auskleidung (NG2-positive Zellen, grün) untersucht. Färbung von Kapillaren wurde mit einem anti-CD31-Antikörper (SC1506, Santa Cruz Biotech, Santa Cruz, USA) und einem Rhodamin-markierten Sekundärantikörper durchgeführt, während die Gefäßreifung durch Perizyten-Kofärbung (anti-NG2-Antikörper AB5320, Millipore, Billerica, USA) quantifiziert wurde. Aufnahmen der ischämischen und nicht-ischämischen Region wurden mit Hochleistungsfeld-Vergrößerung (40fach) gemacht, und 5 unabhängige Bilder pro Region (ischämisch und nicht-ischämisch) und Tier wurden quantifiziert.

### rAA V-Transduktionseffizienz

Für die Auswertung der rAAV-Transduktionseffizienz wurden die Kontrollmäuse, -kaninchen und -schweine mit rAAV.LacZ behandelt. Kryostatschnitte der LacZ-transduzierten Tiere wurden hergestellt und auf β-Galactosidase gefärbt (blaue Färbung). Des Weiteren wurde RT-PCR für die verschiedenen Transgene unter Verwendung der in Tabelle 1 beschriebenen Primer durchgeführt und wie oben beschrieben analysiert.

### Tomato-Reportergen-Mäuse

Diese mT/mG homozygot exprimierenden Mäuse (Jackson Laboratory, Bar Harbor, USA) exprimieren loxP-Stellen an beiden Seiten eines Membran-gerichteten tdTomato (mT) und eines Membran-gerichteten eGFP (Muzumdar et al., Genesis 2007, 45:593-605). Cre-Expression über rAAV.Cre zur Bestimmung der Virustransduktionseffizienz deletierte das mT (rote Fluoreszenz) in den Zellen und ermöglichte eGFP-Expression (grüne Fluoreszenz) in denselben Zellen (Fig. 4b).

### Statistische Verfahren

Die Ergebnisse werden als Mittelwerte ± Standardabweichung angegeben. Statistische Analysen wurden unter Verwendung von Einweg-Varianzanalyse (ANOVA) durchgeführt. Immer wenn ein signifikanter Effekt gefunden wurde (p < 0,05), führte wir mehrfache Vergleichstests zwischen den Gruppen mit dem Student-Newman-Keul-Verfahren durch (IBM SPSS 19.0; IBM, Chicago, USA). Differenzen zwischen Gruppen wurden bei p < 0,05 als signifikant angesehen.

**Tabelle 1**

| Für PCR verwendete Primersequenzen: | | |
|---|---|---|
| BGH vorwärts | 5'-TCT AGT TGC CAG CCA TCT GTT GT-3' | SEQ ID NO:2 |
| BGH rückwärts | 5'-TGG GAG TGG CAC CTT CCA-3' | SEQ ID NO:3 |
| GAPDH vorwärts | 5'-AAT TCA ACG GCA CAG TCA AG-3' | SEQ ID NO:4 |
| GAPDH rückwärts | 5'-ATG GTG GTG AAG ACA CCA GT-3' | SEQ ID NO:5 |
| Tβ4 vorwärts | 5'-TCA TCG ATA TGT CTG ACA AAC-3' | SEQ ID NO:6 |
| Tβ4 rückwärts | 5'-CAG CTT GCT TCT CTT GTT CAA-3' | SEQ ID NO:7 |
| MRTF-A vorwärts | 5'-AAT CCA TGG GTC GAC GGT ATC GAT-3' | SEQ ID NO:8 |
| MRTF-A rückwärts | 5'-ATA CCA TGG TCA GGC ACC GGG CTT-3' | SEQ ID NO:9 |
| CCN1 (CYR61) vorwärts | 5'-GCT AAA CAA CTC AAC GAG GA-3' | SEQ ID NO:10 |
| CCN1 (CYR61) rückwärts | 5'-GGC TGC AAC TGC GCT CCT CTG-3' | SEQ ID NO:11 |
| CCN2 (CTGF) vorwärts | 5'-CCC TAG CTG CCT ACC GAC T-3' | SEQ ID NO:12 |
| CCN2 (CTGF) rückwärts | 5'-CAT TCC ACA GGT CTT AGA ACA GG-3' | SEQ ID NO:13 |
| Ang2 vorwärts | 5'-TCG AAT ACG ATG ACT CGG TG-3' | SEQ ID NO:14 |
| Ang2 rückwärts | 5'-GTT TGT CCC TAT TTC TAT C-3' | SEQ ID NO:15 |

## Patentansprüche

1. Ein Adeno-assoziierter viraler Vektor (AAV-Vektor) umfassend ein Gen, das für einen Myocardin-verwandten Transkriptionsfaktor A (MRTF-A) kodiert.

2. Der AAV-Vektor gemäß Anspruch 1, wobei der AAV-Vektor ein AAV9 oder ein mit Hüllproteinen aus AAV9 pseudotypisierter AAV-Vektor ist, vorzugsweise AAV2.9, AAV1.9 oder AAV6.9.

3. Der AAV-Vektor gemäß irgendeinem der vorhergehenden Ansprüche, wobei das Gen unter der Kontrolle eines kardiospezifischen Promotors steht.

4. Das AAV-Vektor gemäß irgendeinem der Ansprüche 1-2, wobei das Gen unter der Kontrolle eines CMV-Promotors, eines MRC2-Promotors, eines MyoD-Promotors oder eines Troponin-Promotors steht.

5. Eine pharmazeutische Zusammensetzung umfassend einen AAV-Vektor aus irgendeinem der vorhergehenden Ansprüche und einen pharmazeutisch akzeptablen Träger.

6. Ein AAV-Vektor gemäß irgendeinem der Ansprüche 1-4 oder die pharmazeutische Zusammensetzung gemäß Anspruch 5 zur Verwendung als Medikament.

7. Ein AAV-Vektor gemäß irgendeinem der Ansprüche 1-4 oder die pharmazeutische Zusammensetzung gemäß Anspruch 5 zur Verwendung in der Behandlung von koronarer Herzerkrankung oder peripherer Ischämie in einem Säugetier, vorzugsweise in einem Menschen, einer Maus, einem Kaninchen oder einem Schwein.

8. Der AAV-Vektor oder die pharmazeutische Zusammensetzung zur Verwendung gemäß Anspruch 7, wobei die koronare Herzerkrankung akuter Herzinfarkt, myokardiale Ischämie, stabile Angina pectoris und/oder hibernierendes Myokard ist.

9. Der AAV-Vektor oder die pharmazeutische Zusammensetzung zur Verwendung gemäß Anspruch 7, wobei das Säugetier ein menschlicher No-Option-Patient ist.

## Claims

1. An adeno-associated viral vector (AAV vector) comprising a gene encoding a myocardin-related transcription factor A (MRTF-A).

2. The AAV vector according to claim 1, wherein the AAV vector is an AAV9 or an AAV vector pseudotyped with envelope proteins from AAV9, preferably AAV2.9, AAV1.9 or AAV6.9.

3. The AAV vector according to any of the preceding claims, wherein the gene is under control of a cardio-specific promoter.

4. The AAV vector according to any of claims 1-2, wherein the gene is under control of a CMV promoter, a MRC2 promoter, a MyoD promoter or a troponin promoter.

5. A pharmaceutical composition comprising an AAV vector according to any of the preceding claims and a pharmaceutically acceptable carrier.

6. An AAV vector according to any of claims 1-4 or the pharmaceutical composition according to claim 5 for use as a medicament.

7. An AAV vector according to any of claims 1-4 or the pharmaceutical composition according to claim 5 for use in the treatment of coronary heart disease or peripheral ischemia in a mammal, preferably in a human, a mouse, a rabbit or a pig.

8. The AAV vector or the pharmaceutical composition for use according to claim 7, wherein the coronary heart disease is acute myocardial infarction, myocardial ischemia, stable angina pectoris and/or hibernating myocardium.

9. The AAV vector or the pharmaceutical composition for use according to claim 7, wherein the mammal is a human no-option patient.

## Revendications

1. Vecteur viral adéno-associé (vecteur AAV) comprenant un gène codant pour un myocardin-related transcription factor A (MRTF-A).

2. Vecteur AAV selon la revendication 1, dans lequel le vecteur AAV est un AAV9 ou un vecteur AAV pseudotypé avec des protéines d'enveloppe d'AAV9, de préférence AAV2.9, AAV1.9 ou AAV6.9.

3. Vecteur AAV selon l'une quelconque des revendications précédentes, dans lequel le gène est sous le contrôle d'un promoteur cardio-spécifique.

4. Vecteur AAV selon l'une quelconque des revendications 1-2, dans lequel le gène est sous le contrôle d'un promoteur de CMV, d'un promoteur d'MRC2, d'un promoteur de MyoD ou d'un promoteur de troponine.

5. Composition pharmaceutique comprenant un vecteur AAV de l'une quelconque des revendications précédentes et un excipient pharmaceutiquement acceptable.

6. Vecteur AAV selon l'une quelconque des revendications 1 à 4 ou composition pharmaceutique selon la revendication 5 pour son utilisation comme médicament.

7. Vecteur AAV selon l'une quelconque des revendications 1 à 4 ou composition pharmaceutique selon la revendication 5 pour son utilisation dans le traitement de la maladie coronarienne ou de l'ischémie périphérique chez un mammifère, en préférence chez l'homme, la souris, le lapin ou le porc.

8. Vecteur AAV ou composition pharmaceutique pour son utilisation selon la revendication 7, dans laquelle la maladie coronarienne est un infarctus aigu du myocarde, une ischémie myocardique, un angor stable et/ou un myocarde hibernant.

9. Vecteur AAV ou composition pharmaceutique pour son utilisation selon la revendication 7, dans laquelle le mammifère est un patient humain sans option.
